# EUROPEAN PATENT SPECIFICATION

(11) **EP 3 928 814 B1**
(45) Date of publication and mention of the grant of the patent: **14.10.2026**
(21) Application number: 20181604.8
(22) Date of filing: 23.06.2020
(51) Int. Cl.: A61M 5/142, A61M 5/158

(54) **WEARABLE DRUG DELIVERY DEVICE**
TRAGBARE ARZNEIABGABEVORRICHTUNG
DISPOSITIF PORTABLE D'ADMINISTRATION DE MÉDICAMENTS

(43) Date of publication of application: 29.12.2021
(73) Proprietor: mylife Diabetes Care AG, 3400 Burgdorf (CH)
(72) Inventor: Baumert, Jan, 3455 Grünen (CH); Hanimann, Michael, 3012 Bern (CH); Margot, Roland, 3079 Worb (CH); Streit, Ursina, 3322 Schönbühl (CH); Bosshard, Simon, 3006 Bern (CH); Steiner, Fabian, 3400 Burgdorf (CH)
(74) Representative: Finklenburg, Susanne

(56) References cited:
- EP-A1- 2 762 183
- EP-A1- 3 598 942
- WO-A2-2016/130679
- US-A1- 2004 064 096
- US-A1- 2007 282 269
- US-A1- 2013 060 233

## Description

### FIELD OF THE INVENTION

The present invention relates to a drug delivery device, in particular to a wearable patch pump for subcutaneous delivery of a fluid medicament from a reservoir. Advantageous variants of the invention include an improved design or an improved method of manufacturing a drug delivery device.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include drug delivery devices that are removed from the injection site after each medication event or drug delivery process, as well as infusion devices with a cannula or needle that remains in the skin of the patient for a prolonged period of time. By way of example, diabetes may be treated by administration of insulin by the patients themselves with the help of multi-variable-dose insulin injection pens or infusion pumps. Alternatively, patch injectors, wearable injectors or wearable pumps are patched or adhered to the skin of the patient.

Departing from classical syringes, increasingly complex devices have been designed to support different therapies, to ensure safety and reliability, and to increase ease of use to a point patients can apply the drugs themselves, reducing time-consuming and costly interventions by trained medical staff to a minimum. Examples of drug delivery devices suitable for self-treatment include injection pens, auto-injectors, portable infusion pumps and wearable patch pumps. Despite of the technical complexity it is an important requirement to keep cost of manufacturing and cost of devices as low as possible.

Common to all devices for subcutaneous drug delivery is a reservoir to store the fluid medicament, and a fluid path to bring the drug out of the device and into the subcutaneous tissue of a patient. Fluid-tightness of the fluid path is an essential requirement to ensure safety and accuracy of the delivery. Longer term infusion patterns and reliable system supervision functions particularly rely on controlled fluid pressure along the fluid path. While this is rather easy to achieve for a classic syringe, it becomes a challenge with increasing complexity of the device. Requirements are further increased by design for self-administration, which means use in a non-sterile environment, use by people without medical training, or even use by people with reduced visual or haptic capacities. The use of pre-filled cartridges, user-friendly fill ports, modular devices with disposable modules as well as wearable devices with auto-inserters are typical solutions to improve ease of use. Reducing the number of mechanical components and design for easy assembly during manufacturing are typical approaches to minimise cost.

US 6,669,668 B1 discloses a drug delivery device with a disposable reservoir and a reusable pump module. The drug is manually filled into the disposable reservoir using a standard syringe. An administration set is used to bring the drug from the pump into the body of the patient.

An important step towards ease of use is to omit the administration set and design a wearable patch pump which is small and has an adhesive patch to attach the pump to the patient during drug delivery. A typical patch pump design has a housing with a reservoir to contain the drug, a cannula to lead the drug into the body of a patient, and a needle assembly to establish a fluid-tight connection between the reservoir and the cannula. For optimum ease of use, the cannula is made of a soft material and an auto-inserting mechanism with a rigid needle or cannula is built into the pump to insert the soft cannula into the body of the patient for drug delivery. For compact and fluid-tight design, the reservoir is generally built into the housing and needs to be filled from outside prior to use. A number of sealing components are needed to ensure a fluid-tight design of the fluid path and of the housing. Special solutions are needed for the fill port, where the drug is brought into the reservoir, and for the exit port, where the cannula passes from the inside of the housing to the outside of the housing for drug delivery. Wearability asks for a compact design of the patch pump as a whole, which further adds to the complexity of design and manufacturing. As the most complex variation of subcutaneous drug delivery devices, semi-disposable patch pumps with internal auto-inserting mechanism and a soft cannula open the door to the most sophisticated therapies at the highest level of ease of use at a potentially low cost. Among other applications, they are a preferred solution for the intermittent delivery of insulin for the treatment of diabetes mellitus.

There is clearly a strong need for a wearable drug delivery device which provides accurate and reliable drug delivery in a compact, easy to use, fluid-tight and robust design and which can be manufactured at low cost. To arrive at an optimal solution, all involved components have to be designed accordingly.

US 7,303,549 B2 describes a fully disposable patch pump for transcutaneous fluid delivery. While this concept includes an auto-inserting mechanism for the injection needle and offers a high level of ease of use, the lack of reusable parts brings the disadvantage of generating a lot of waste and increased cost of the therapy.

US 8,679,062 B2 describes a semi-disposable patch pump describes a modular patch pump where one of the modules is reusable to reduce waste. However, ease of use is affected by the requirement to handle several different modules and by lacking the auto-inserting mechanism for the injection needle.

US 9,993,595 B2 describes another example of a modular patch pump in a more compact design. Again, the missing auto-inserting mechanism affects ease of use.

For wearable delivery devices a compact design is of particular importance. The material used for such wearable drug delivery devices is usually plastic due to its advantageous manufacturing characteristics and its low density.

In other technical fields different types of material are often combined to profit from advantageous characteristics from different materials. For example, US 5,597,990 B1 discloses an electrical switch integrated in a switch-box and designed for detecting the presence of an electronic memory card in a card reader device. The housing of the switch box carries two fixed electrical contact elements which are arranged laterally on either side of the housing. Each fixed contact element is made of a folded metal blade whose free ends project out of the switch box to constitute terminals for connection and soldering on a printed circuit board. The contact elements are partially embedded into an overmoulded plastic housing of the switch box.

In WO03103763 a patch injection device is disclosed comprising an external housing for containing a reservoir. The reservoir is closed by a needle insertion septum and may be filled by an external filling means using a fill port located in the housing.

In WO2017120251 a filling assist mechanism for a patch pump is disclosed comprising features to allow easier and more reliable filling of a reservoir located inside the patch pump. The reservoir is closed by a needle insertion septum and may be filled through a fill port located in the housing of the pump. The external filling assist mechanism comprises a cone shaped opening for guiding the needle of an external filling device. The very existence of the filling assist documents that the fill port as far as integrated in the patch pump is cumbersome to use.

US 2019/0091404 A1 discloses a cartridge-based drug delivery device where the reservoir has a sealing membrane at the outlet. To connect the reservoir outlet to the needle assembly and prepare the pump for drug delivery the membrane is pierced by the needle assembly. In a compact design such a membrane can be difficult to manufacture.

US 6,699,218 B2 describes a patch pump with a soft cannula and a rigid cannula slidably moving axially in the soft cannula for insertion. All connections along the fluid path are designed to prevent leaks, but no solution is given how to design the interface between the two cannulas for fluid-tightness at a specified occlusion pressure. Fluid-tightness at a specified occlusion pressure is necessary for reliable detection of an occlusions in the fluid path.

The most common approach to an exit port sealing is by piercing a septum. One such arrangement is disclosed in EP 1390089 B1 as part of an infusion set. The more complex design of a patch pump with auto-inserting mechanism opens additional possibilities for new solutions by extending this basic concept and by optimising the exit port assembly for the specific requirements of the application.

EP 3598942 A1 discloses a brute force approach, where the insertion mechanism simply drives the cannula through the housing wall.

In US 2007/282269 A1 an exit port septum is provided to close the exit port opening prior to use. The needle assembly is driven through the exit port septum for insertion into the body of the patient.

US 2004/064096 A1 is an example of a more sophisticated design, where a septum or extra sealing is pierced by a cannula when inserting said cannula into the body of a patient. The main sealing between cannula and surrounding components is provided by the inserting mechanism which moves together with the cannula. This approach has advantages for that particular sealing function to the cost of adding complexity to the exit port assembly.

In US 2013/060233 A1, and also in WO 2016/130679 A2, a whole flexible boot is provided between the insertion mechanism holding the cannula and the housing. Again, the result is an exit port assembly with a high number of components and high complexity in manufacturing.

EP 2762183 A1 discloses an alternative exit port design, where the angle of the cannula for insertion into the body of the patient can be adjusted. The tip of the cannula remains in a flexible sealing. This approach avoids the piercing of a sealing during insertion of the cannula into the body of a patient, but adds complexity to the manufacturing process because it makes needle guidance inside the pump housing more difficult. Further, the sealing function of the flexible sealing is compromised by the extra adjusting function.

An exit port assembly for a patch pump with soft cannula and inserting mechanism is disclosed in EP 1682203 B1. The assembly as disclosed includes a multitude of sealing components, making it more costly and less than optimal for manufacturing.

US 7,771,412 B2 describes an environmental seal for a fluid delivery device where the exit port is designed as a cap which comprises two different components and is mounted into a housing. Although 2-shot moulding is mentioned as a way to improve manufacturability and reduce cost, the proposed exit port is rather complex. The soft plug remains in the inside of the cap and is not arranged in a way allowing combination with other functions for further optimisation.

In a patch pump with auto-inserting mechanism a fluid connection between the reservoir and the output of the cannula is established when manufacturing the pump. This open path can affect the accuracy of drug delivery or also the function of the filling process. It is therefore desirable to close this path until the drug delivery is intended to start. An objective is to provide a solution for the closure of the exit port which is easy to use and suitable for low cost manufacturing.

US 7,018,360 B2 discloses a semi-permeable exit plug to support filling and priming a patch pump with needle inserting mechanism. The plug may be a sheet attached to the adhesive release liner of the adhesive patch. Although simple, this concept is prone to tearing the semi-permeable sheet in the process of removal of the plug and asks for a solution with improved reliability and hence improved ease of use.

US 6,749,587 B2 discloses a patch pump where the adhesive is provided in a continuous ring encircling the exit port assembly to provide a protective seal around the penetrated skin. Again, this simple concept can be extended to include other functions and find an overall optimum.

EP 3251585 A1 discloses an adhesive patch assembly for a patch pump, including structures to improve reliability of the connection with the body of the patient by letting air and humidity pass from the surface of the skin to the environment. This concept can also be extended and combined with other functions to achieve an optimum of reliability and ease of use.

It is an objective of the invention to provide an improved drug delivery device which is accurate, reliable, easy to use and cost effective, overcoming the drawbacks of or introducing alternatives to the prior art. Several aspects of the present invention contribute to an overall optimum. They are all applicable to all sorts of drug delivery devices such as pen injectors, patch injectors, mobile pumps or patch pumps.

It is an additional objective to provide improved assembly methods for the drug delivery device as disclosed in the corresponding claims.

### DEFINITIONS

The term "substance", "drug", "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the needle configured to penetrate the skin of the patient. For an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours. If not explicitly mentioned otherwise, the term "pump" is referring to an infusion system, in the context of the present invention typically to a patch pump.

In the claims, the word "comprising" does not exclude other elements or steps, and the indefinite article "a" or "an" does not exclude a plurality. For example "sealing" or "protrusion" does not exclude the fact that there may be two "sealings" or "protrusions" that functionally or structurally fulfil the purpose of "a sealing" or "a protrusion".

### DESCRIPTION OF THE INVENTION

In a first aspect of the present invention, a modular concept for a semi-disposable patch pump is introduced, providing an optimal compromise between ease of use and re-use of components. This is achieved by reducing the number of components the user has to deal with to two: the reservoir unit, intended for single use, and the pump unit, intended for multiple or continuous use. The fact that there are just the two units minimises waste while still minimising the number of handling steps the patient needs to perform to apply the pump.

The reservoir unit comprises a reservoir unit housing and a base plate mounted on an adhesive patch, with the base plate being attached to or integrated into the bottom of the reservoir unit housing, and includes all components intended for single use, in particular all components in contact with the drug and the adhesive patch. Drug carrying components will typically include a reservoir with a reservoir outlet, a pump mechanism, and a needle assembly connected to the outlet of the reservoir to transport the drug from the reservoir into the body of the patient. For optimum ease of use, the reservoir unit further includes an inserter assembly which includes an auto-inserting mechanism to insert the needle into the body of the patient without any force being applied by the user. The pump mechanism can for example comprise a plunger movably mounted in the reservoir, allowing the drug to be pressed out of the reservoir through the reservoir outlet by moving the plunger. Any kind of energy can be used to drive the auto-inserting mechanism, either energy stored in the reservoir unit like a pre-loaded insertion spring or energy originating from outside like a rotation generated by the pump unit. This also includes the possibility of using the same drive unit to drive or release the auto-inserter as used for driving the pump mechanism. The pump unit includes all components intended for multiple or continuous use, for example a pump unit housing, an electrical power source like a rechargeable battery, a drive mechanism with a driving means to drive the pump mechanism in the reservoir unit, acoustic and/or visual and/or haptic elements to interact with the user, a communication unit to send and/or receive data to/from other system components, and a control unit to control the device. In an embodiment where the pump mechanism is a plunger movably mounted in the reservoir, the driving means could for example be a combination of a motor, gearing and coupling to a threaded rod in cooperation with a plunger rod.

The pump unit is further configured to be releasably attachable to the reservoir unit. It is an important aspect of the present invention how the reservoir unit and the pump unit are mechanically connected. The connection has to be safe and reliable, allowing easy separation of the pump unit and the reservoir unit for replacement of the disposable parts, while ensuring optimum safety and reliability when it comes to unintended separation or removal of the pump unit during normal use.

The patch pump, with both units connected, preferably forms a substantially edge-free shape for best wearability and to avoid unintended removal of the patch pump from the body of the patient. The outer shape enveloping the pump components during normal use also contributes to avoiding unintended detaching of the pump unit from the reservoir unit. Still aiming for optimum ease of use, the mechanical interface between the reservoir unit and the pump unit preferably includes a bayonet coupling, where no button needs to be pressed or slider needs to be moved to release the connection, while still ensuring a mechanical connection with optimum rigidity and minimal axial play as required for accurate and reliable drug delivery.

The pump unit is simply pushed onto the reservoir unit at an angle of at least 5 degrees, preferable at least 15 degrees and folded down onto the plane of the base plate, thereby closing the substantially edge-free enveloping outer surface of the pump. The closing movement of the reservoir unit is substantially a rotation around an axis defined by the bayonet connection. To open the bayonet, the user will pull on the side of the reusable unit facing away from the rotation axis of the bayonet, where the pump unit is lifted off the plane of the base plate to release the pump unit.

While the concept of the semi-disposable patch pump as described in the present invention includes any kind of locking mechanism for the bayonet connection, mechanisms allowing the same handling step to unlock and open the connection are particularly easy to use and consequently preferred. Examples for this kind of embodiments are a mechanical latch snapping in and out by simple pushing and pulling, a magnetic lock or a pair of Velcro^{®} strips.

Although the enveloping outer shape of the patch pump is optimised for wearability, unlocking and detaching the pump unit may still occur if the body part of the patient where the pump is attached inadvertently brushes over an edge. The edge may slide under the patch pump on the opening side, causing the pump unit to be lifted off the plane of the base plate. The locking mechanism may open and the pump unit may be brushed off the body of the patient. To avoid this kind of unintended detaching, an improved embodiment of the patch pump comprises a base plate which is extended from the bottom of the reservoir unit housing between the opening side and the bayonet axis in the direction of the pump unit. While this extended base plate efficiently reduces the risk of unintended detaching of the pump unit from the reservoir unit, it could make it more difficult for the patient to remove the pump unit intentionally. Therefore, a further improvement to the patch pump according to the present invention is a cut-out at the edge of the base plate. The effect of the cut-out is that a finger of the patient can at least partially slide under the pump unit on the opening side of the pump, while a longer edge is still kept outside. To achieve this effect, the cut-out would have a length of 5 mm to 30 mm, preferably 15 to 25 mm and a width of 1 mm to 10 mm, preferably 3 mm to 5 mm.

The patch pump according to the present invention reduces the number of handling steps required to apply the pump to a minimum, while also minimising the waste generated by the therapy. Additional features make sure the patch pump is safe to use despite its ease of use.

**In** a second aspect of the present invention, a concept for a compact design of a drug delivery device is introduced. This objective is achieved by a drug delivery device with a reservoir for holding a liquid drug, a needle assembly for delivery of the drug, and a base frame.

According to the second aspect of the present invention the drug delivery device comprises a base frame comprising an electrically conductive connector structure including electrical contact areas for establishing an electrical connection between the base frame and an external device. The base frame further comprises a non-conductive body injection-moulded around the connector structure such that the electrical contact areas are contactable by the external device from outside the non-conductive body. The base frame may further be integrated into or form a part of the housing of the drug delivery device.

The present aspect of the invention may further include the base frame itself for a drug delivery device as described in the previous paragraph, the base frame comprising an electrically conductive connector structure including electrical contact areas for establishing an electrical connection and a non-conductive body injection-moulded around the connector structure such that the electrical contact areas are contactable from outside the non-conductive body, characterised in that the non-conductive body includes a guiding member for guiding a movement of the needle assembly, combined with the non-conductive body to form an unitary component.

According to prior art approaches different electrical contact elements and a support for holding the contact elements in place inside the drug delivery are separately mechanically connected to each other, for example by heat staking, by riveting, by insertion or pressing into sockets, or by using snap lock connections. The different electrical elements are complex to handle. Furthermore, establishing an electrical connection between the different elements requires a considerable number of manufacturing steps.

According to the second aspect of the present invention the base frame as a support structure allows to considerably reduce the number of single components and also allows to combine several functions in one assembly. The electrical connector structure comprises electrical conductors, contacts, tracks or strip lines adapted to establish an electrical connection between electrical components supported or held by the non-conductive body. The connector structure may be formed by only one component or a small number of components. The non-conductive body of the drug delivery device is preferably injection-moulded around the connector structure. This concept allows to integrate various other supporting elements into the non-conductive body, forming a unitary component with extended functionality. Other supporting elements may be, for example, supporting pins for a printed circuit board, retaining elements for retaining a battery on the non-conductive body, guiding elements for guiding a moveable needle assembly or other moveable elements of the inserter mechanism, or stop elements to stop such a movement. By reducing the number of components the base frame according to the present invention allows a compact design of the drug delivery device.

The electrically conductive parts or connector members of the connector structure may be made of any electrically conductive material such as metal, metal alloys, conductive plastics or a conductive composite material. The connector structure provides an electrical connection between the components supported by the base frame and external devices, which are inseparably or releasably connected to the electrical contact areas of the base frame. Furthermore, the connector structure may provide electrical connections between various electrical and/or electronic components supported or guided by the base frame.

The connector structure may comprise only one single conductive part or connector member or it may comprise multiple conductive parts or connector members. Preferably, the connector structure comprises at least two separate connector members which are electrically insulated from each other. In this case, all connector members of at least one connector structure are preferably manufactured as one single component in a first production step, for example one single sheet of metal. The shape of the sheet will include all connector members plus some connecting bridges between them to form one single conductive component. In a second production step the conductive sheet or component is overmolded, forming one unitary component with a non-conducting frame holding all connector members as well as the connectinig bridges. In a third production step the connecting bridges are removed, for example by blanking, resulting in a unitary component containing a connector structure with one or more separated electrical pathways. This manufacturing process is considerably easier and more time efficient than using the overmoulding technology with two or more conductive connector members all inlaid as separate components.

The electrical contact areas are integrated into the connector structure to form one unitary component. However, as mentioned above, the connector structure itself may comprise more than one conductive part or connector member. Preferably, a first electrical contact area is formed in a first connector member and a second electrical contact area is formed in a second connector member, wherein the first and the second electrical contact area are galvanically distinct and separated from each other. In this case the first electrical contact area is preferably adapted to be connected to a positive pole and the second electrical contact area is adapted to be connected to a negative pole.

The non-conductive body of the drug delivery device is injection-moulded around the connector structure and is thus at least partially enveloping the connector structure. The connector structure is therefore supported or held by the non-conductive body. This allows to securely place the connector structure, for example, inside a housing of the drug delivery device. The non-conductive body may be made of electrically non-conductive plastic, non-conductive composite material or any other non-conductive injection-mouldable material.

The non-conductive body is preferably one unitary component. Several elements such as, for example, retaining elements, guiding tracks, mechanical stop elements, supporting elements or bearing pins are formed integrally in the non-conductive body. That means the elements are preferably made of the same material as the non-conductive body.

The non-conductive body is injection-moulded around the connector structure such that the electrical contact areas of the connector structure are contactable from outside the non-conductive body. That means that the non-conductive body is designed not to prevent the electrical contact areas of the connector structure from establishing contact with the intended connecting contact. The electrical contact areas of the connector structure may be designed, for example, in form of arms or levers protruding out of the non-conductive body or the non-conductive body may have an opening or a recess allowing an external device to be contacted with the electrical contact areas through the opening or recess.

The connector structure and the non-conductive body injection-moulded around the connector structure may form a hybrid component or a unitary component manufactured using two-component (2C) moulding technology. This technology is also known as insert moulding or overmoulding technology.

The drug delivery device may be an infusion system or an injection system. The drug delivery device may be, for example, an injection pen or it may be a patch injector applicable onto the skin of the user for the duration of the injection. The infusion system may be, for example, a conventional medical drug pump such as an insulin pump with an infusion set, or it may be a drug patch pump without infusion set and attachable directly onto the skin of the user.

In preferred embodiments the drug delivery device is a patch pump comprising a reusable part and a disposable part. The reusable part may comprise a drive mechanism and a control unit. The disposable part is adapted to be replaced after each infusion and it may comprise a reservoir with the drug, a hybrid assembly with the needle assembly, a cannula moving assembly to move at least a portion of the needle assembly, and the base frame according to the invention. **In** this constellation, the cannula moving assembly would comprise relevant parts of the inserter assembly dealing with the movement of the needle assembly, in particular holders for a rigid cannula and for a soft cannula.

In a preferred embodiment the non-conductive body forms a guiding member for guiding a movement of the needle assembly itself or the cannula moving assembly. The term "forms" means the guiding member is integrally formed in the non-conductive body in one unitary component. Thus, the guiding member is made of the same material as the rest of the non-conductive body.

The guiding member may guide the moveable portion of the needle assembly or the cannula moving assembly along a linear or straight movement path, along a curved path, along a rotational path or along a combination of linear, curved and rotational movement path. The guiding member may be realised, for example, in form of a rail, a nut, a protrusion or a profile.

Since the guiding member is formed in the non-conductive body as one unitary component no means for connecting the guiding member to the non-conductive body are required, e.g. no snap or screw connection is needed. The guiding member allows an easy and quick movement of the cannula and/or other portion of the needle assembly, for example, from a start or initial position to an extended or inserted position, where the cannula is inserted into the skin of the user.

Preferably, the guiding member is a linear guiding rail for guiding the needle assembly itself or a portion of the cannula moving assembly along a linear movement path. "Linear" means the movement path is a straight line such that the needle assembly shifts along a straight line. The guiding member may comprise one or more guiding rails. Preferably, the guiding element comprises at least one guiding rail which is adapted to engage with a corresponding counterpart, for example a groove, of the needle assembly and/or the cannula moving assembly.

Advantageously, the non-conductive body may comprise an end stop surface integrated in the non-conductive body as an unitary component and wherein the end stop surface is adapted to restrict a movement of the needle assembly or the cannula moving assembly in a first direction. The end stop surface is integrally formed in the non-conductive body. Preferably, the end stop surface defines either an extended position or an initial position of the needle assembly. The needle assembly is moveable from an initial or start position to the extended or inserted position, where the cannula has pierced the skin of the user. The end stop surface may be, for example, a bump stop, a buffer or just an element protruding in the direction of movement of the needle assembly.

Preferably, the connector structure is mainly made of metal. That means the connector structure is either completely made of metal or made of metal and other material components, conductive composite materials or made of metal and additional non-metal materials. Preferably, the connector structure is made of one electrical conductive metal sheet. That allows an easy and efficient production of the connector structure.

In this case the connector structure is preferably made by blanking and bending. That means the contours of the connector structure are defined in a first step by blanking the connector structure from a metal sheet and in a second step the connector structure is further formed by bending. If the connector structure comprises more than one connector member the connector members are initially mechanically connected to each other via auxiliary or temporary bridges or connectors. These bridges are eliminated or interrupted by a punching step after the non-conductive body has been injection-moulded around the circuit. That means that the finished connector structure may comprise several galvanically distinct and separated connector members or other electrical pathways. In this way, the connector structure can be produced efficiently and at lower cost.

In a preferred example the connector structure comprises at least two electrical contact areas. Preferably, each of the at least two electrical contact areas is arranged on a resilient element or contacting arm of a connector member. That means the connector structure comprises at least two connector members each comprising a resilient element or contacting arm carrying an electrical contact area. The electrical contact areas can thus bounce or flex when an external element mechanically and electrically contacts the electrical contact areas. That may help to reliably establish an electrical connection between the external contact element and the electrical contact areas of the connector structure. An external contact element may belong to an other device like a charger, or may belong to any other system component connectable to the connector structure like a unit of a modular pump or an add-on for monitoring an injection or infusion process.

Preferably, the drug delivery device comprises a switching arm protruding flexibly outside the base frame and formed in a first connector member in one unitary component. Hence, the switching arm is made of the same material as the connector structure. The switching arm is preferably directly or indirectly electrically contactable with a second connector member. The first and the second connector member are galvanically separated from each other. If the switching arm electrically contacts the second connector member an electrical connection is established between the first and second connector member and thus an electrical signal can be transmitted. Hence, the switching arm can be switched by the movable cannula moving assembly between a conductive state and a non-conductive state.

The generated signal may indicate, for example, whether or not a release button is pressed, an external device is connected or the needle assembly is moved into a specific position. Of course, the switching arm has to be positioned such that the button, the external device or the needle assembly may mechanically contact the switching arm to switch the lever between the conductive state and the non-conductive state.

Advantageously, the switching arm is switchable by the needle assembly or by the inserter assembly such that the switching arm establishes an electrical connection or interrupts an existing electrical connection between the first connector member and the second connector member.

The switching arm is advantageously positioned next or near the movement path of the cannula moving assembly or the needle assembly such that an element (for example, an edge, a protrusion, an actuating lever) of the cannula moving assembly or the needle assembly may mechanically contact and thus switch the switching arm. The conductive state (electrical connection) or the non-conductive state (connection electrically interrupted) of the switching arm may be indicative that the needle assembly is in an initial position or in an extended position or that it is just not anymore in the initial or extended position.

In a preferred embodiment the non-conductive body may mainly be made of plastic. The non-conductive body may comprise only plastic or mainly plastic and other materials or composite material, which is electrically non-conductive. In either case, the material of the non-conductive body is injection-mouldable and can thus be used for overmoulding the connector structure.

Further, the non-conductive body may comprise a retaining element for retaining a battery integrated into the non-conductive body as one unitary component. Furthermore, the connector structure may comprise battery contact elements for electrically contacting the retained battery integrated into the connector structure as one unitary component. The retaining element may be, for example, an arm, a clamp, a retaining tab or a tongue. The battery can thus be reliably held relative to the non-conductive body. The battery may be a button cell, a conventional cylindrical battery or an accumulator. If the battery is retained and held in place by the retaining element the battery mechanically and electrically contacts the battery contact elements of the connector structure. The battery contact elements may be realised, for example, in form of pins, tabs, tongues or arms and may be integrated into the connector structure as one unitary component.

Preferably, the connector structure forms at least two battery contact elements. To electrically contact the battery a first battery contact element is adapted to be connected to the positive pole and a second battery contact element is adapted to be connected to the negative pole of the battery.

The invention relates further to a base frame for a drug delivery device with a needle assembly, preferably also an inserter assembly with a cannula moving assembly. The base frame comprises an electrically conductive connector structure including electrical contact areas for establishing an electrical connection and a non-conductive body injection-moulded around the connector structure such that the electrical contact areas are contactable from outside the non-conductive body. The non-conductive body includes a guiding member for guiding a movement of the needle assembly or the cannula moving assembly, integrated into the non-conductive body as one unitary component. Furthermore, the invention relates to a hybrid assembly comprising the above described base frame, the cannula moving assembly and the needle assembly.

In a preferred example the hybrid assembly further comprises a heater assembly or a melt release module with a heating element for releasing a piercing process with the needle assembly. The heater assembly is supported by the non-conductive body.

The releasing of the piercing process may involve a linear or rotational movement of the needle assembly from an initial or start position to an extended position, where the cannula has pierced the skin of the user. The heating element of the heater assembly preferably melts or breaks a fuse, which holds the biased needle assembly or cannula moving assembly in its initial position. If the fuse is melted or broken an insertion trigger or retaining means of the hybrid assembly breaks or expands such that the biased needle assembly or cannula moving assembly is free to move the cannula and to pierce the skin of the user with a portion of the needle assembly.

The heater assembly with the heating element is preferably directly or indirectly via a further element supported by the non-conductive body and arranged on said non-conductive body.

For controlling the insertion process the hybrid assembly preferably comprises a printed circuit board (Reservoir unit printed circuit board, PCB-RU) supported and held by the non-conductive body. The heating element of the heater assembly and other controlling elements may be arranged on the PCB-RU.

Preferably, a holding structure for rigidly holding the PCB-RU on the non-conductive body is integrated into the non-conductive body as one unitary component. The structure may comprise pins, tabs or arms formed as one unitary component. The PCB-RU may be fixed to holding structure, for example, by an adhesive, by heat staking or by a snap lock.

The design of a sealing for a drug delivery device is not an isolated task. Fluid-tightness is always defined for a specific area or volume, according to pressure requirements derived from the intended functionality. To arrive at the design of the current invention, the following pressure requirements have been specified:
- to allow successful filling of the reservoir through a fill port in the housing, at least the reservoir sealing and the fill port sealing are fluid-tight to a filling pressure of at least 6 bar
- to allow successful occlusion detection, at least the sealing of the fluid path of the medicament is fluid-tight to an occlusion pressure of at least 2 bar
- to allow successful protection from ingress from the environment, at least the sealing of the housing is fluid-tight to an environmental overpressure of at least 0.24 bar.

It is particularly important to fulfil these requirements and find an overall optimum for sealing at all interfaces relevant for the function of the drug delivery device. It is further evident that the values of the pressure requirements given above are just examples to explain the advantages of the present invention, and shall by no means restrict the application of the concepts described in this invention to a specific pressure range.

In a third aspect of the present invention, a concept for an improved fill port assembly is described overcoming the drawbacks of the prior art. The improved fill port assembly ensures fluid-tightness at filling pressure, facilitates easy assembly, enhances the longevity and robustness of the device, and may additionally fixate the reservoir with respect to the housing.

This objective is solved by a drug delivery device for delivery of a medicament from a reservoir to a patient, comprising: a housing comprising a wall separating an interior volume from the exterior, a reservoir being located in the interior volume, a fill port assembly located in the wall of the housing and accessible from the exterior for filling the reservoir, the fill port assembly comprising a cone shaped opening for receiving a needle and a pierceable fill port sealing separating the reservoir from the exterior, characterised by that the fill port assembly comprises an insert adapted to be received by a passage in the wall of the housing and wherein the fill port sealing fulfils a sealing function between the housing and the reservoir. The sealing between the housing and the reservoir is provided to make sure no fluid may pass between the reservoir and the wall of the housing.

The current aspect of the invention is preferably applied to a drug delivery device for delivery of a medicament from a reservoir, the medicament preferably being a liquid or a solid that is reconstituted prior to the injection. The reservoir may be a cartridge or non-collapsible reservoir or collapsible reservoir made from a flexible material. The drug delivery device comprises a housing comprising a wall separating an interior volume from the exterior. The housing may be constructed in any number of components connected together to form a substantially closed shell around the interior volume. The reservoir is located in the interior volume. A fill port assembly is located in, or is part of, the wall forming the housing and is accessible from the exterior for filling the reservoir. The reservoir may be filled at the factory or the user fills the reservoir prior to use. Preferably, the reservoir is a multiple use reservoir. Optionally, the reservoir is filled only once and the unit comprising the reservoir is discarded after use. The fill port assembly comprises a cone or conical shaped opening adapted to receive a needle. The cone shaped opening facilitates the entry of the needle of a filling or transfer device, for example a syringe, containing the medicament for filling an empty reservoir with the medicament. The fill port assembly comprises a pierceable fill port sealing separating the reservoir from the exterior. The pierceable fill port sealing may comprise a pierceable septum. The fill port sealing or the septum of the sealing may be pierced by the needle for filling the reservoir. Furthermore, the fill port assembly comprises an insert which is adapted to be received by a passage in the wall of the housing or to be received in the fill port sealing whereby the latter is received in the passage of the housing. The fill port assembly is assembled from exterior of the housing. The fill port sealing provides a sealing between the housing and the reservoir. The fill port sealing may provide a plurality of sealings to different sections or components within the drug delivery device and the sealing may be a direct sealing between the housing and the reservoir or an indirect sealing via other housing parts. Combining multiple sealings in the fill port assembly may reduce the number of parts needed. Using an insert as part of the fill port assembly has the advantage that during assembly the insert is inserted from the outside into the passage in the wall thereby also possibly fixating other parts (for example housing parts) or components such as the reservoir with respect to at least part of the housing of the drug delivery device or the reservoir unit of the drug delivery device and simultaneously establishing the sealing or plurality of sealings. Thus the fill port assembly combines the features of enabling the filling of an empty reservoir, sealing of the reservoir with respect to the housing and fixation of the cartridge or reservoir. The use of an insert separate from the housing has the advantage that the two parts can be adapted to their specific needs. The insert may be made from a different material than the housing and thus reduce manufacturing costs or made of a material having a different stiffness or wear resistance, with the effect of reducing the risk of particle abrasion, needle clogging or needle damage. The cone shaped opening further facilitates the needle insertion through the fill port, especially for the visually impaired users.

The insert of the drug delivery device preferably comprises the cone shaped opening and the cone shaped opening extends from a base. The cone shaped opening defines a longitudinal axis and the base is preferably connected to the larger diameter access of the cone and is oriented perpendicular to the longitudinal axis. The outer dimensions of the base fit into a first recess or recessed section of the housing or the wall of the housing. The cone shaped opening of the insert is thus adapted to receive and guide the needle tip, for example towards the septum of the fill port sealing. The cone shaped opening has an angle with respect to the longitudinal axis. The opening of the cone is wide to facilitate access of the needle tip. The cone angle between the longitudinal axis and the cone surface ranges between 20 and 40 degrees, more preferably between 25 and 35 degrees, most preferably the cone angle is approximately 30 degrees.

The fill port sealing is preferably sandwiched between the base of the insert and the wall of the housing to provide a first sealing between the insert and the housing. .The first sealing prevents leakage of fluids and/or gases from inside the housing to the exterior, or reversed contamination from the exterior entering into the housing. The first sealing may be part of a sterility concept to protect parts inside the housing from the ambient. The first sealing may enhance the shelf life or longevity of the device.

The fill port sealing comprises a flange or a rim which is preferably made from compressible or elastic material such as an elastomer. The flange is adapted to be received in a second recess or recessed section surrounding the passage in the wall of the housing. The first recessed section for the insert may be different from the second recessed section for the flange of the fill port sealing. Both recessed sections surround the passage and the second recessed section for the flange may be deeper compared to the first recessed section for the insert. The second recessed section for the flange is adapted to receive the flange or rim and preferably has a lateral dimension smaller than the lateral dimensions of the first recessed section adapted to receive the base of the insert. Preferably the flange is sandwiched between the insert (or the base of the insert) and the wall. The first sealing is preferably an axial sealing oriented along the longitudinal axis of the conical shaped opening and located between the base of the insert and the housing or the recessed section surrounding the passage of the housing. Reception of the flange of the fill port sealing in the second recessed section in the wall secures the fill port sealing from lateral movement and aligns the parts, e.g. passage in the housing, fill port sealing and insert prior to final assembly and/or fixation of the cartridge. The wall of the housing in the second recessed section configured to receive the fill port sealing and/or the base of the insert facing the fill port sealing may have protrusions or protruding structures to locally compress the resilient sealing material of the flange.

The drug delivery device may further be improved by a fill port additionally sealing an inlet of the reservoir. The inlet of the reservoir is preferably oriented parallel to the longitudinal axis of the fill port assembly (e.g. parallel to the longitudinal axis of the cone shaped opening) and the inlet of the reservoir is aligned with respect to the passage in the housing once the device has been assembled. The inlet of the reservoir is preferably oriented perpendicular to the longitudinal axis of the reservoir. The inlet of the reservoir is also aligned with respect to the cone shaped opening and the septum that is part of the fill port sealing such that penetration of the septum by the needle ensures that medicament can be filled into the reservoir.. The fluid port assembly thus provides a fluid tight closure of the reservoir, preferably of the inlet of the reservoir and thus preventing leakage of medicament from the reservoir into the housing or to the exterior. The fluid port assembly also prevents contamination from the exterior entering the reservoir and may be part of a sterile barrier.

The fill port sealing of the drug delivery device may provide a second sealing between the fill port sealing and the inlet of the reservoir and the sealing is preferably oriented in a radial direction perpendicular to the longitudinal axis of the cone shaped opening. The second sealing is preferably axially displaced from the first sealing. Two sealing functions in one part are enabled by locating the two sealings at different locations, both having (independent) sealing properties and the material or dimensions may be adapted to the specific needs.

Preferably, the insert and the fill port sealing fixate the reservoir with respect to the housing. Thus by inserting the fill port sealing and insert from the outside into the housing, the reservoir is fixated which is preferably already present inside the housing. The fixation may be based on a form-fit fixation, alternatively using a friction or force fit. The fixation may additionally benefit from a snap-fit connection between the reservoir, or the inlet of the reservoir and at least one of the parts forming the fill port assembly . The fixation preferably uses the fill port assembly or parts thereof being at least partially located inside the inlet of the reservoir. Alternatively a neck section or the body of the reservoir is used for the fixation. Using the fill port assembly additionally for reservoir fixation implies that sealing and fixation are combined which improves the assembly of the device and reduces parts.

Preferably, the fill port sealing comprises a bore extending from the flange and ending in the pierceable septum. The bore may be formed by a cylindrical section connecting the pierceable septum to the flange of the fill port sealing. Preferably, the pierceable septum forms the end of the bore. The cylindrical section preferably connects the first and second sealings.

Preferably the insert of the drug delivery device comprises a sleeve extending from the cone shaped opening, starting from the narrow section of the cone shaped opening and the sleeve is adapted to be received within the bore of the fill port sealing. The cone thus may connect the base to the sleeve. The sleeve is connected to the narrow end of the cone shaped opening and preferably extends along the longitudinal axis of the cone. The sleeve guides the needle tip from the cone shaped opening towards the pierceable septum of the fill port sealing. The guidance ensures that the septum is pierced perpendicular to a membrane forming the septum to prevent tearing of the septum and leakage along the needle during filling of the reservoir. The guidance also prevents the needle from getting in contact with the inner wall of the inlet, which would bear the risk of abrading particles from the inlet wall and of reducing the depth of piercing. Furthermore, the sleeve of the insert is coaxially arranged within the bore formed by the cylindrical section of the fill port sealing. The sleeve of the insert, the cylindrical section of the sealing and the inlet of the reservoir may be coaxially arranged for insertion of the insert and sealing during assembly and for reservoir fixation of the assembled device. The reservoir may be fixated by mounting the fill port assembly since the base of the insert is fixated in at least one of the recessed sections of the housing and prevents lateral movement of both the fill port sealing and the reservoir.

Preferably, an outside surface of the fill port sealing or an outside surface of the cylindrical section is at least partially received in the inlet of the reservoir.

The outside surface of the cylindrical section is preferably press fitted into the inlet of the reservoir-thereby a) establishing the second sealing (due to resilience or partial resilience of outside surface of the cylindrical section) and b) fixating the reservoir from lateral movement. The fill port assembly may fixate the reservoir such that there is a shock absorber between the reservoir and the housing, preferably due to the elastic properties of the outside surface of the cylindrical section engaging the inlet of the reservoir. This is advantageous for the device as it improves the impact resistance, for example during a drop test.

The insert for the drug delivery device is made from a metal such as steel, preferably stainless steel or aluminium. Alternatively, the insert is made from a plastic material coated with a metal layer or from a plastic material that is strengthened with a mineral filler such as glass, zirconia or aluminium oxide particles. One advantage of using a metal or a toughened plastic is to prevent the needle tip from abrading particles from the insert or even getting stuck in the insert. Another advantage of using a metal or a toughened plastic is to improve the wear resistance of the insert to an extent that the reservoir may be used multiple times, e.g. a needle is guided and inserted through the fill port assembly multiple times without damaging the insert. Therefore the longevity of the insert, and therewith the device, particularly for a re-usable device may be improved.

The fill port sealing preferably comprises a thermoplastic polymer and an elastomer. Preferably, the elastomer surrounds the thermoplastic polymer. The advantage is that the thermoplastic polymer has a higher modulus for providing the mechanical strength for fixation of the reservoir, while at the same time improving suitability for assembly during manufacturing of the pump. The advantage of using the elastomer is that the elasticity is beneficial for forming the sealings and optionally for the shock absorber. Examples for the thermoplastic polymers may be polybutylene terephthalate (PBT), polycarbonate or polycarbonate alloys such as cycoloy, acrylonitrile butadiene styrene copolymers (ABS) or a high modulus polyurethane (PUR). Examples for the elastomer may by an EPDM rubber, polydimethylsiloxane rubber PDMS, preferably in LSR form or an elastomeric polyurethane (PUR) or a thermoplastic elastomer (TPE). The thermoplastic polymers and elastomers are preferably selected to fulfil the biocompatibility requirements according to ISO 10993 as parts of the fill port sealing may be in contact with the medicament. The thermoplastic polymer and elastomer are preferably injection moulded using 2-component injection moulding, thereby reducing the number of parts as a plurality of functions are combined in a single part.

The insert of the fill port assembly, more preferably the base of the insert comprises a recess or an opening or cut-out configured to receive a fixation pin extending from the wall of the housing. The insert may comprise a plurality of recesses, openings or cut-outs adapted to engage a plurality of fixation pins. The fixation pins are preferably integrally formed on the housing and made from a thermoplastic polymer. The fixation pins are oriented substantially parallel to the cone axis and the engagement between the cut-outs and the fixation pins ensure that after mounting the insert is correctly aligned with the other parts of the fill port assembly and the housing. The engagement between the fixation pins and the openings facilitate the assembly and ensure that the insert is rotationally fixed with respect to the housing. The insert may be fixed to the housing by heat staking of at least one of the fixation pins e.g. by heating and deforming a fixation pin. This axial and rotational fixation is fundamental for reliable and sufficient contact pressure of the fill port sealing and hence for achieving the intended sealing function of the fill port up to the specified filling pressure.

The drug delivery according to this aspect of the current invention allows the assembly of the fill port from exterior of the housing, and uses a separate insert for fixation of the septum and of the reservoir. The insert may be made from a different material compared to the housing material.

The current aspect of the invention also includes providing an improved method for assembling the drug delivery device with a fill port according to the description above, comprising the steps of:
- providing the housing, the reservoir, the fill port sealing and the insert
- inserting the reservoir from the exterior into the housing along an axis that is perpendicular to the cone axis, followed by
- inserting the fill port sealing from the exterior along the cone axis into the passage 211c in the wall of the housing, followed by
- inserting the insert from the outside in the fill port sealing thereby sandwiching the fill port sealing (preferably the flange of the fill port sealing) between the insert (preferably the base of the insert) and the housing and establishing the first and second sealings and fixate the reservoir to the housing.

The method may additionally comprise the following step:
Positioning the opening or cut-out of the insert on the fixation pin extending from the wall of the housing of the drug delivery device or the disposable unit, followed by heat staking of the fixation pin to fixate the insert to the housing. Alternatively the insert may be fixated to the housing using ultrasonic welding, laser welding, using an adhesive or heat welding.

In a 4^{th} aspect of the present invention, a concept for an improved sealing of a drug delivery device is described at the interface of the reservoir and the needle assembly leading the drug out of the reservoir towards the cannula. The reservoir outlet sealing design ensures fluid-tightness at occlusion pressure, facilitates easy assembly and enhances the longevity and robustness of the device.

This objective is solved by a drug delivery device comprising a housing with a reservoir located inside the housing to contain a drug; a needle assembly with an input portion and an output portion; and a pierceable reservoir outlet seal; whereby the reservoir comprises an at least partially rigid reservoir housing with an at least partially cylindrical reservoir outlet sealing cavity, integrated into the reservoir housing as a unitary component; the reservoir outlet sealing is at least partially cylindrical and configured to be inserted into the reservoir outlet sealing cavity during manufacturing of the patch pump, to be pierced by the input portion of the needle assembly during manufacturing, and to form a fluid-tight connection between the reservoir and the input portion of the needle assembly. The at least partially cylindrical design of the reservoir outlet sealing contributes to ensuring fluid-tightness at the filling and/or occlusion pressure and to an easier assembly process compared to other geometries. In a variation of this design, the soft reservoir outlet sealing may be manufactured together with the rigid reservoir housing using 2-shot injection moulding as a unitary component.

In a 5^{th} aspect of the present invention, a concept for an improved sealing of a drug delivery device is described at the interface of the rigid cannula as part of the input portion of the needle assembly, and the soft cannula as part of the output portion of the needle assembly. The soft cannula input sealing design ensures fluid-tightness at occlusion pressure, facilitates easy assembly and enhances the longevity and robustness of the device.

This objective is solved by a drug delivery device comprising a housing with a reservoir located inside the housing to contain a drug; a needle assembly with an input portion and an output portion; whereby the needle assembly comprises a soft cannula having an open distal end, and a rigid cannula at least partially and slidably disposed in an inner soft cannula lumen of the soft cannula; the proximal input end of the rigid cannula is part of the input portion of the needle assembly and the distal output end of the soft cannula is part of the output portion of the needle assembly; whereby the proximal input end of the soft cannula forms a sliding soft cannula input sealing configured to slide on the rigid cannula while applying a tightening pressure to ensure a fluid-tight sealing at the occlusion pressure.

In a 6^{th} aspect of the present invention, a concept for an improved sealing of a drug delivery device is described at the interface of the housing and the output portion of the needle assembly. The improved exit port sealing ensures fluid-tightness of the housing at environmental pressure, reduces the number of components of the drug delivery device, facilitates easy assembly, saves cost and enhances the longevity and robustness of the device.

This objective is solved by a drug delivery device comprising a housing with an enveloping surface, separating an interior volume from the exterior; a reservoir located inside the housing to contain a drug; a needle assembly with an input portion and an output portion; and an exit port assembly, whereby the housing comprises at least two components, configured to be attached to one another to form a protective shell of the drug delivery device; the reservoir comprises a reservoir outlet coupled to the input portion of the needle assembly; the housing comprises an exit port opening to provide a passage for the output portion of the needle assembly from the interior of the housing to the exterior; the exit port assembly is configured to form a fluid-tight connection between the housing and the output portion of the needle assembly; the exit port assembly comprises a rigid exit port sealing holder with an at least partially tubular exit port channel defining an exit port channel axis configured to receive the output portion of the needle assembly, and a soft exit port sealing attached to the rigid exit port sealing holder during manufacturing of the drug delivery device; the soft exit port sealing is configured to be pierced by the output portion of the needle assembly during manufacturing of the drug delivery device; *and whereby* the soft exit port sealing is further configured to provide a fluid-tight closure of the exit port opening of the housing when the exit port assembly is mounted on the housing.

Three exemplary approaches to realise this concept are explicitly described in the following paragraphs.

One exit port sealing design according to the concept as described is realised by using a 2-shot injection moulding process to manufacture the exit port assembly as a unitary component comprising the rigid exit port sealing holder and the soft exit port sealing.

Another exit port sealing design according to the concept as described is realised by joining the exit port assembly together with one of the at least two housing components and by using a 2-shot injection moulding process to manufacture the exit port assembly as a unitary component comprising at least one of the at least two housing components, the rigid exit port sealing holder, and the soft exit port sealing. By including a part of the housing, this multi-functional component may particularly include other sealings of the housing, such as the sealing at the mechanical interface between the reservoir unit and the pump unit of a semi-disposable patch pump.

A further exit port sealing design according to the concept as described is realised by adapting the rigid exit port sealing holder to further include an exit port sealing plug cavity defining an exit port sealing plug cavity axis, and open on at least one end to receive the soft exit port sealing; the exit port sealing plug cavity is arranged to intersect the exit port channel with an angle of at least 10 degrees, preferably at least 45 degrees between the exit port channel axis and the exit port sealing plug cavity axis; the exit port sealing plug cavity axis and the exit port channel axis intersect at an angle of at least 10 degrees, preferably at least 45 degrees; the soft exit port sealing is inserted into the exit port sealing plug cavity during manufacturing of the drug delivery; and the soft exit port sealing is configured to tightly close the exit port sealing plug cavity and the exit port opening when mounted in the rigid exit port sealing holder.
to the input portion of the needle assembly; the housing comprises an exit port opening to provide a passage for the output portion of the needle assembly from the interior of the housing to the exterior; the exit port assembly is configured to form a fluid-tight connection between the housing and the output portion of the needle assembly; the exit port assembly comprises a rigid exit port sealing holder with an at least partially tubular exit port channel defining an exit port channel axis configured to receive the output portion of the needle assembly, and a soft exit port sealing attached to the rigid exit port sealing holder; the soft exit port sealingis configured to be movable from an initial position for storage to an inserted position for drug delivery; the exit port assembly is fixedly attached to at least one component of the housing; *and whereby* the soft exit port sealing is further configured to provide a fluid-tight closure of the exit port opening of the housing with the output portion of the needle assembly located on the distal side of the soft exit port sealing both in the initial position and in the inserted position of the needle assembly.

Three exemplary approaches to realise this concept are explicitly described in the following paragraphs.

One exit port sealing design according to the concept as described is realised by using a 2-shot injection moulding process to manufacture the exit port assembly as a unitary component comprising the rigid exit port sealing holder and the soft exit port sealing.

Another exit port sealing design according to the concept as described is realised by joining the exit port assembly together with one of the at least two housing components and by using a 2-shot injection moulding process to manufacture the exit port assembly as a unitary component comprising at least one of the at least two housing components, the rigid exit port sealing holder, and the soft exit port sealing. By including a part of the housing, this multi-functional component may particularly include other sealings of the housing, such as the sealing at the mechanical interface between the reservoir unit and the pump unit of a semi-disposable patch pump.

A further exit port sealing design according to the concept as described is realised by adapting the rigid exit port sealing holder to further include an exit port sealing plug cavity defining an exit port sealing plug cavity axis, and open on at least one end to receive the soft exit port sealing; the exit port sealing plug cavity is arranged to intersect the exit port channel with an angle of at least 10 degrees, preferably at least 45 degrees between the exit port channel axis and the exit port sealing plug cavity axis; the exit port sealing plug cavity axis and the exit port channel axis intersect at an angle of at least 10 degrees, preferably at least 45 degrees; the soft exit port sealing is inserted into the exit port sealing plug cavity during manufacturing of the drug delivery; and the soft exit port sealing is configured to tightly close the exit port sealing plug cavity and the exit port opening when mounted in the rigid exit port sealing holder.

A further advantageous feature of the exit port sealing design according to the concept as described is realised by modifying the exit port sealing plug cavity to include a constriction and/or flattening at the exit port opening to improve pressing and/or shaping of the soft exit port sealing at the intersection with the exit port channel. Increased pressing brings fluid-tightness at a higher pressure, a flat shape of the exit port sealing is easier to pierce during manufacturing.

A further advantageous feature of the exit port sealing design according to the concept as described is realised by modifying, in one of the devices described before, the shape of the housing, and by introducing a recess from the enveloping surface of the housing at the exit port opening. With the exit port located in a recess, the patch pump can be manufactured ready to use with the output portion of the needle assembly already going through the exit port, providing adequate sealing and ease of use without having any part protruding from the enveloping surface of the housing. Further, the recess in the housing forms an external exit port chamber between the recessed exit port opening and the enveloping surface of the housing, which allows filling of the reservoir and filling of the fluid path prior to use without risk of injecting any medicament into the body of the patient. By filling the fluid path with medicament before inserting the cannula into the body of the patient and before starting the drug delivery the pump avoids infusing air instead of medicament at the beginning of the infusion process and improves the accuracy of drug delivery.

The features of the exit port sealing design according to the concept as described, regardless of the approach for realisation, are especially advantageous to a semi-disposable patch pump with a needle assembly, whereby the needle assembly comprises a soft cannula having an open distal end, a rigid cannula at least partially and slidably disposed in an inner soft cannula lumen of the soft cannula, and a needle insertion mechanism configured to bring the output portion of the needle assembly with at a least the open distal end of the soft cannula from a first position inside the exit port chamber to a second position outside the exit port chamber in the exterior of the housing. Such an insertion mechanism substantially improves ease of use.

In a 7^{th} aspect of the present invention, a concept for an improved sealing of a patch pump is described at the interface of the housing and the environment in the area of the exit port prior to the application of the pump to the body of the patient. The exit port lid design ensures fluid-tightness at a minimal filling pressure as specified for the use of the patch pump, supports the process of filling and priming and facilitates easy assembly and use of the device.

This objective is solved by a drug delivery device with an exit port located in a recess of the housing - like for example the device described in the previous aspect of the invention - and an exit port lid, whereby the exit port lid is removably attached to the housing at a portion of the housing surrounding the exit port opening; the exit port lid covers the exit port chamber in a way that prevents fluid from entering or leaving the exit port chamber while letting air pass; the exit port lid includes a membrane of any semi-permeable material adapted to allow air to pass through while stopping liquid from passing; the exit port lid includes a membrane reinforcing structure permanently fixed to the membrane to ensure the membrane is not damaged in the process of removing the exit port lid from the housing. Such a membrane reinforcing structure may be a plastic sheet with a cut-out for the exit port opening.

**In** a 8^{th} aspect of the present invention, a concept for an improved sealing of a patch pump is described at the interface of the housing and the environment in the area of the exit port after attaching the pump to the body of the patient. The design of the housing and the design of the adhesive patch assembly improve the reliability and robustness of drug delivery, while also providing an easier and more comfortable use of the patch pump.

This objective is solved by a drug delivery device as described before and used as a patch pump with an adhesive patch assembly to attach the pump to a patient, whereby the adhesive patch assembly comprises an adhesive layer configured to attach the adhesive patch assembly to the patient after preparation of the patch pump for drug delivery; the adhesive patch assembly comprises a removable adhesive release liner to protect the adhesive layer from unintended adhering prior to use, in particular during preparation of the patch pump for drug delivery; the exit port lid is fixed permanently to the adhesive release liner and removably connected to the housing; and the exit port lid is configured to be removed from the housing together with the adhesive release liner during preparation of the patch pump for drug delivery.

The effect of the design of the housing and the adhesive patch assembly in the area of the exit port is especially advantageous for a patch pump as described before, whereby the housing further comprises a membrane carrying structure arranged around the exit port chamber; the membrane carrying structure is adapted to protrude from the housing towards the exterior by a height substantially the same as the thickness of the adhesive patch assembly; the membrane carrying structure is configured to provide contact with the exit port lid in a status where the exit port lid is removably attached to the housing; the membrane carrying structure is configured to provide a fluid-tight connection between the exit port lid and the housing during preparation of the patch pump for drug delivery and thereby tightly close the exit port chamber.

While a reliable attachment of the adhesive patch assembly to the body of the patient, in particular in the area of the exit port, is important for the reliability of the drug delivery, a further improvement of the patch pump design at the interface of the housing and the environment in the area of the exit port is to introduce, at a distance from the exit port, a number of airing channels. They allow air and/or humidity leave the interface and improve the adherence of the patch pump to the body of the patient during drug delivery. This is realised with a patch pump as described before, whereby the housing and/or the adhesive patch assembly further comprises at least one ventilation structure with at least one inner end closed and arranged at a horizontal distance of 1 mm to 20 mm, preferably 5 mm to 10 mm from the exit port chamber, and at least one outer end left open and arranged at the peripheral edge of the housing and/or the adhesive patch. The horizontal distance of the airing channel from the exit port chamber is important to maintain a tight closure around the exit port and avoid a connection of the ventilation structure with the exit port chamber. The ventilation structure is configured to let air pass from the inner end to the outer end and to the environment of the patch pump. During drug delivery, the airing channel formed by the ventilation structure may be on the surface of the body of the patient - if the ventilation structure is realised in the adhesive patch assembly - or more towards the housing - if the ventilation structure is formed by the housing or by an other layer of the adhesive patch assembly.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of a patch pump as seen from above (away from patient, left) and from below (towards the patient, right);
- Fig. 2a,b: refer to the mechanical interface between the pump unit and the reservoir unit;

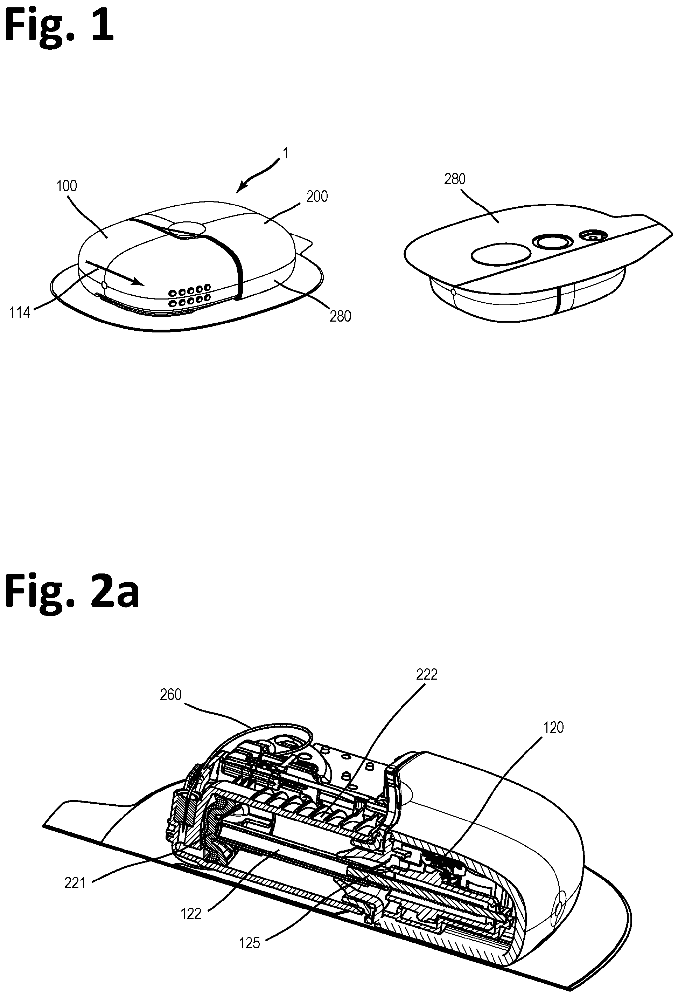

Fig. 2a depicts the drive mechanism of the patch pump across the two units;

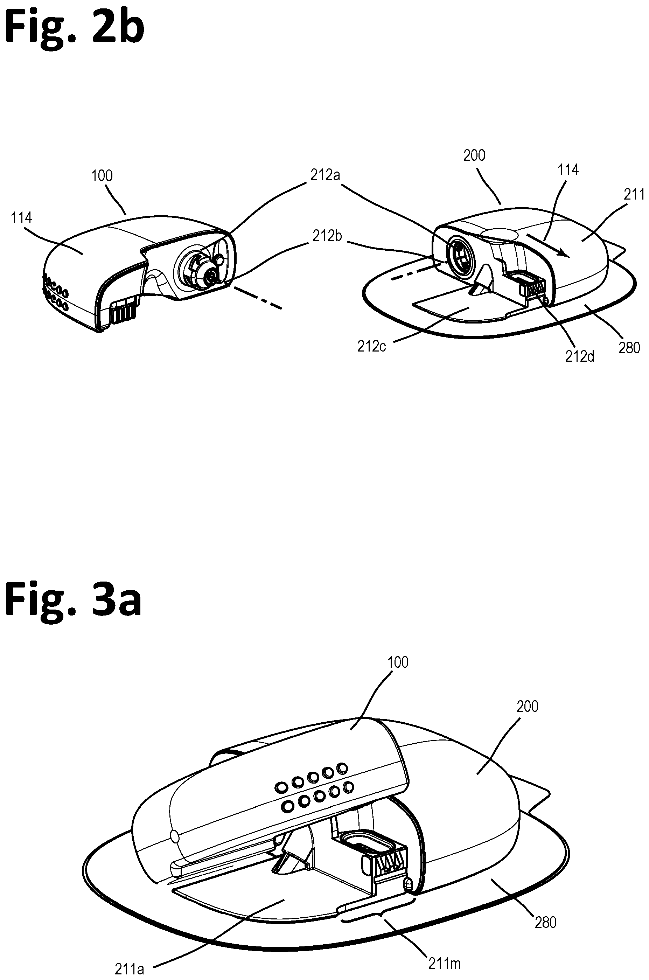

Fig. 2b depicts the bayonet connection between the pump unit and the reservoir unit of a patch pump;

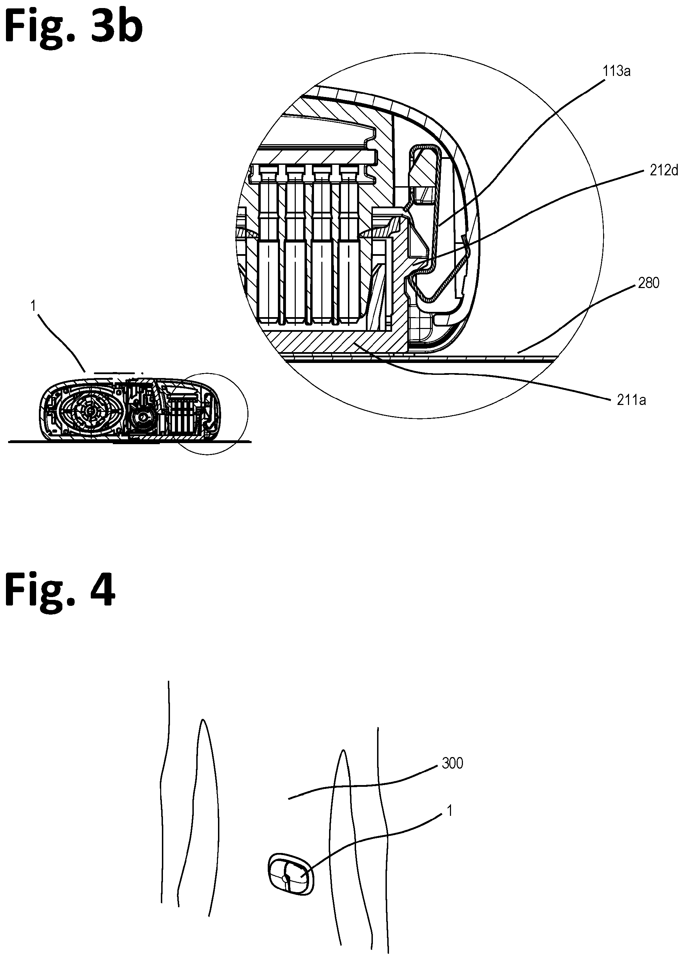

- Fig. 3a,b: refer to the step of attaching the Pump Unit to the Reservoir Unit of a patch pump;
Fig. 3a depicts the patch pump with the pump module connecting the bayonet of the reservoir module at an angle of 30°;
Fig. 3b depicts a cut across the patch pump at the locking mechanism, with locking mechanism closed (left: complete cut, right: enlarged view of the locking mechanism);
- Fig. 4: depicts the patch pump attached to the body of a patient and ready for drug delivery;

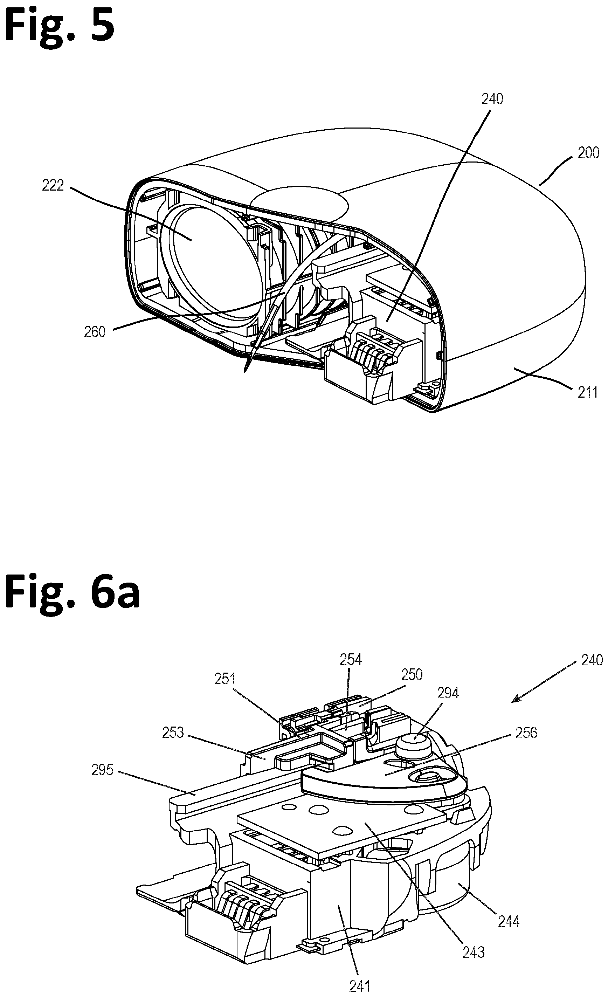

- Fig. 5: depicts the reservoir unit of the patch pump without the front side of the housing and the adhesive release liner;

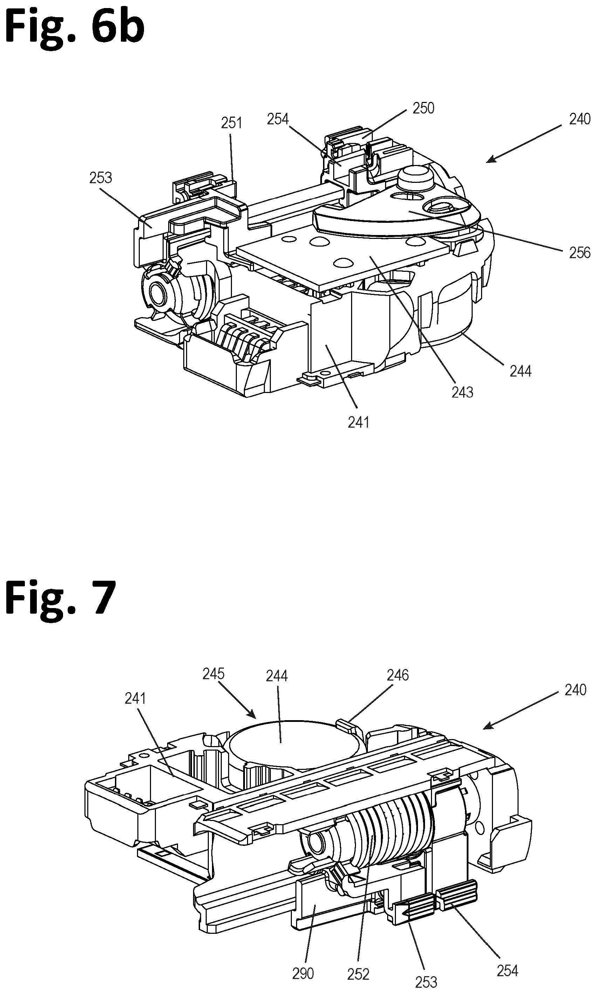

- Fig. 6a,b: refer to a perspective view of the hybrid assembly with the cannula moving assembly;
Fig. 6a depicts a perspective view of the hybrid assembly with the cannula moving assembly in the initial position;
Fig. 6b depicts the hybrid assembly of figure 6a but with the cannula moving assembly in the extended position;
- Fig. 7: depicts a bottom view of the hybrid assembly of figure 6a,b;

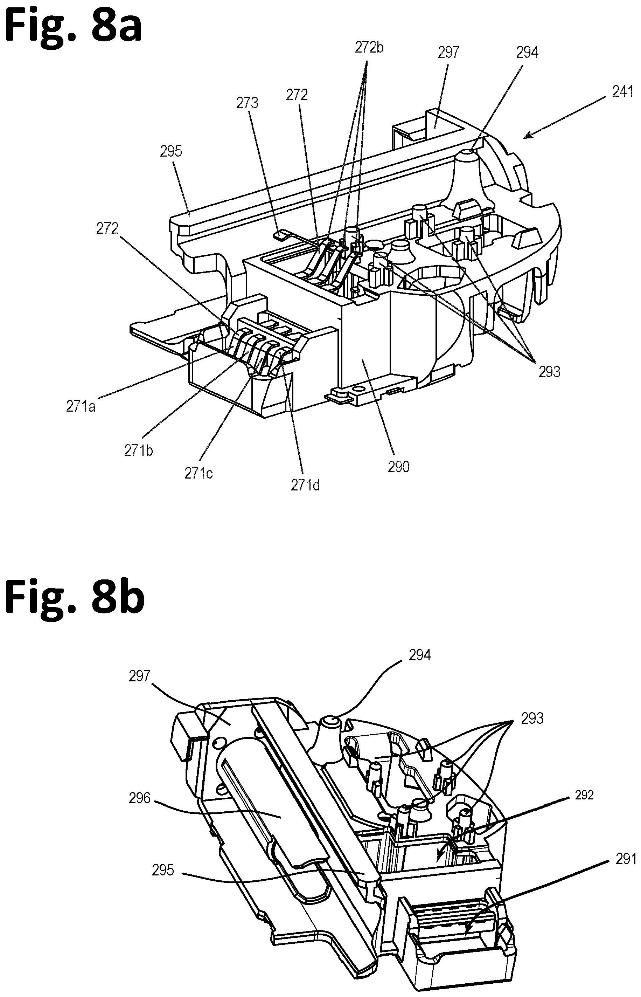

- Fig. 8a-c: refer to a perspective view of the base frame;
Fig. 8a depicts a perspective view of the base frame comprising a non-conductive body and a connector structure;
Fig. 8b depicts a perspective view of the non-conductive body of the base frame, bottom-up;

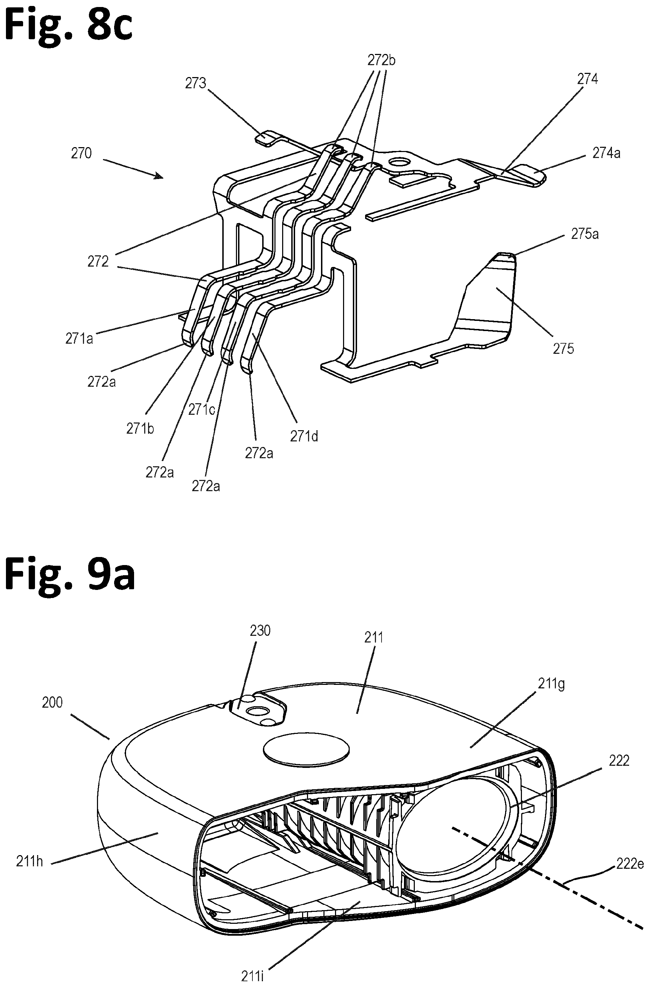

Fig. 8c depicts the connector structure of the base frame.

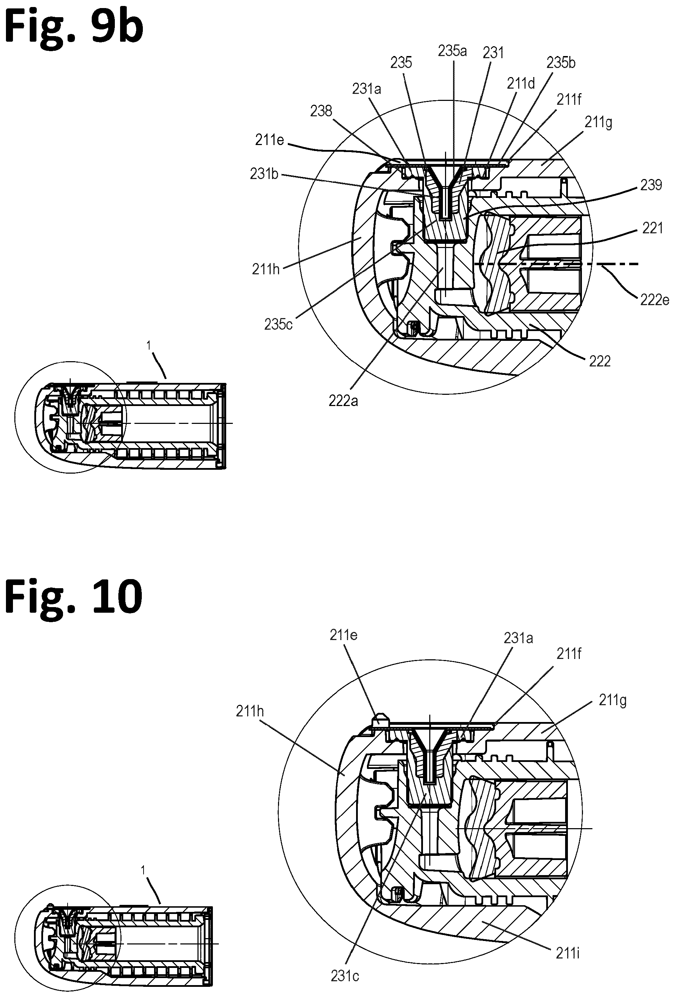

- Fig. 9a,b: refer to a perspective view of the reservoir unit with a fill port, after fixation by hot stemming;
Fig. 9a depicts a perspective view of a reservoir unit, partially assembled and turned bottom up to show the fill port at the bottom of the unit;
Fig. 9b depicts a cross section of the reservoir unit with fixated fill port assembly and reservoir inlet;
- Fig. 10: depicts the cross section of a reservoir unit from figure 9a,b as seen before fixation of the fill port by heat staking;

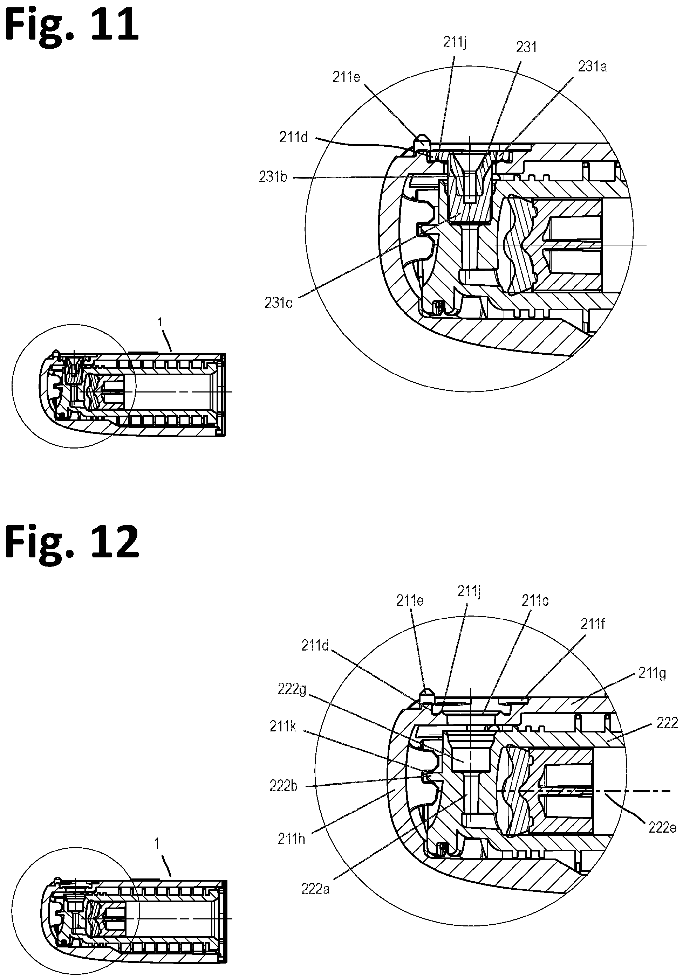

- Fig. 11: depicts a cross section of the reservoir unit from figure 9a,b with inserted fill port sealing, without the insert;
- Fig. 12: depicts a cross section of the reservoir unit from figure 9a,b before mounting the fill port assembly;

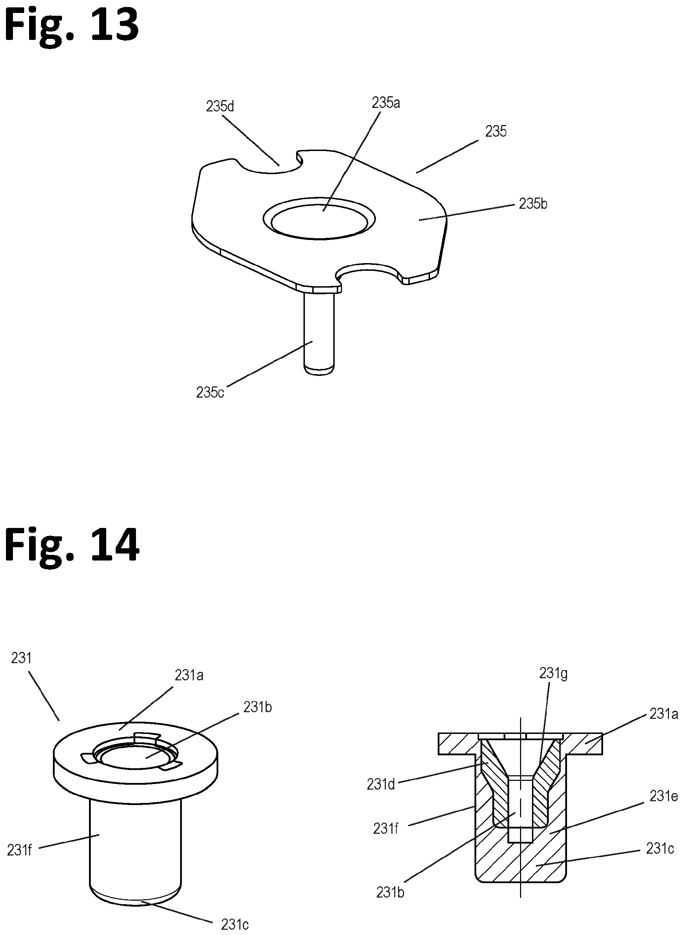

- Fig. 13: depicts a perspective view of the insert;
- Fig. 14: depicts the fill port sealing in a perspective view (left) and a cross section (right);

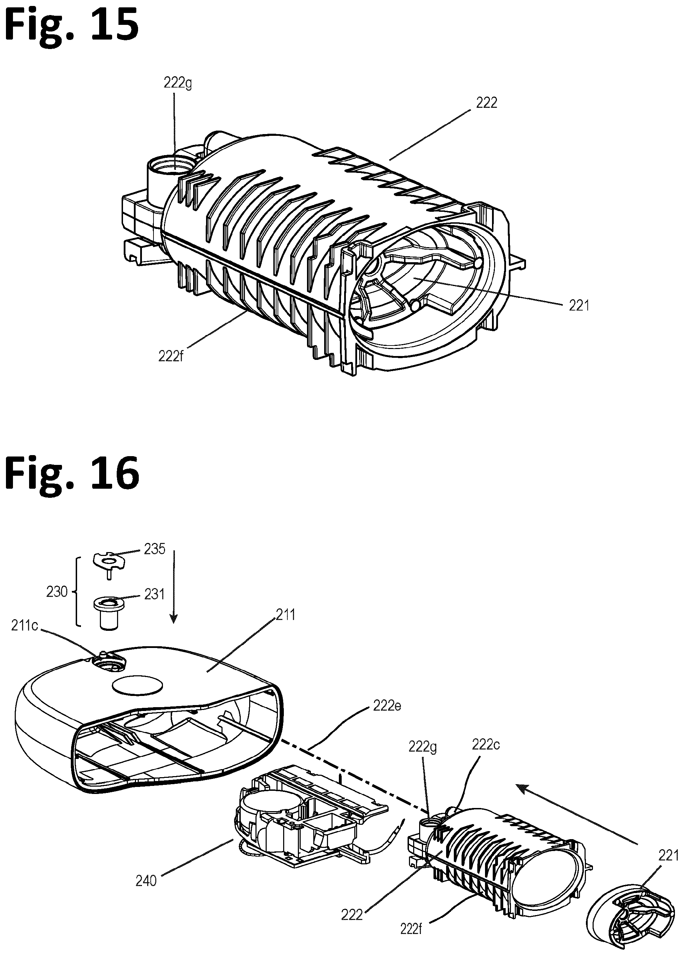

- Fig. 15: depicts a perspective view of a reservoir including plunger;
- Fig. 16: depicts an exploded view of the reservoir unit showing the insertion of the cartridge and of the fill port assembly into the reservoir unit housing to form the reservoir unit;

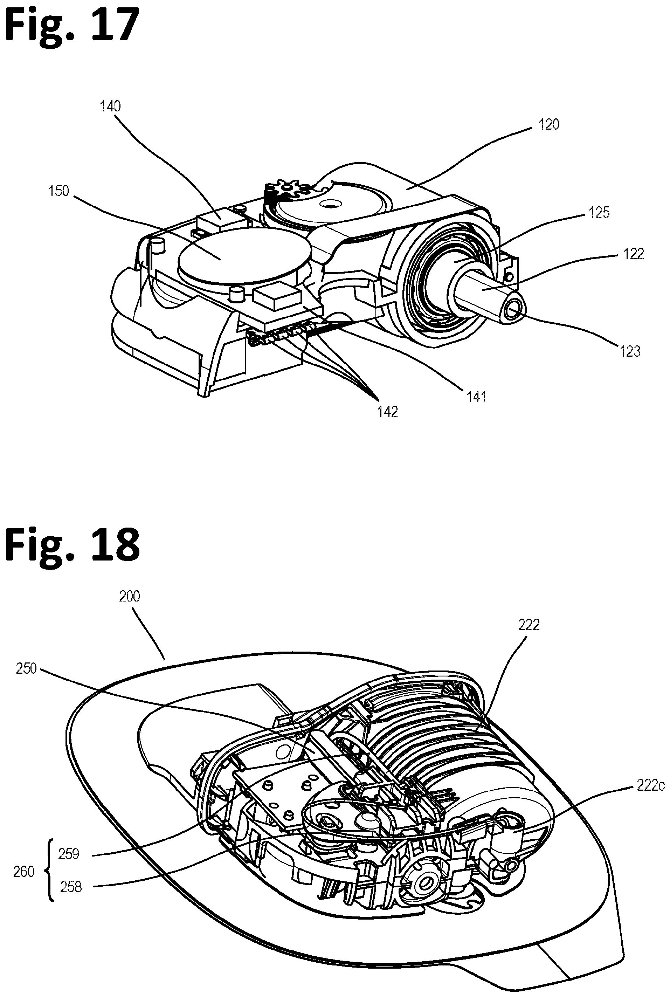

- Fig. 17: depicts a perspective view of a pump unit with housing removed;
- Fig. 18: depicts a perspective view of a reservoir unit with part of the housing removed

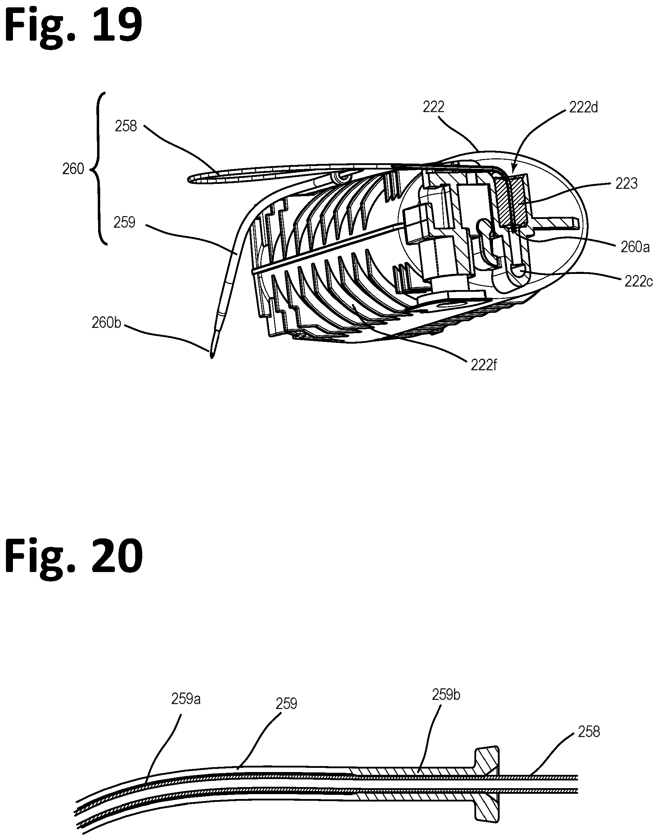

- Fig. 19: depicts a patch pump with a reservoir outlet seal, cut across the reservoir outlet
- Fig. 20: depicts a cut across a patch pump with a soft cannula input sealing;

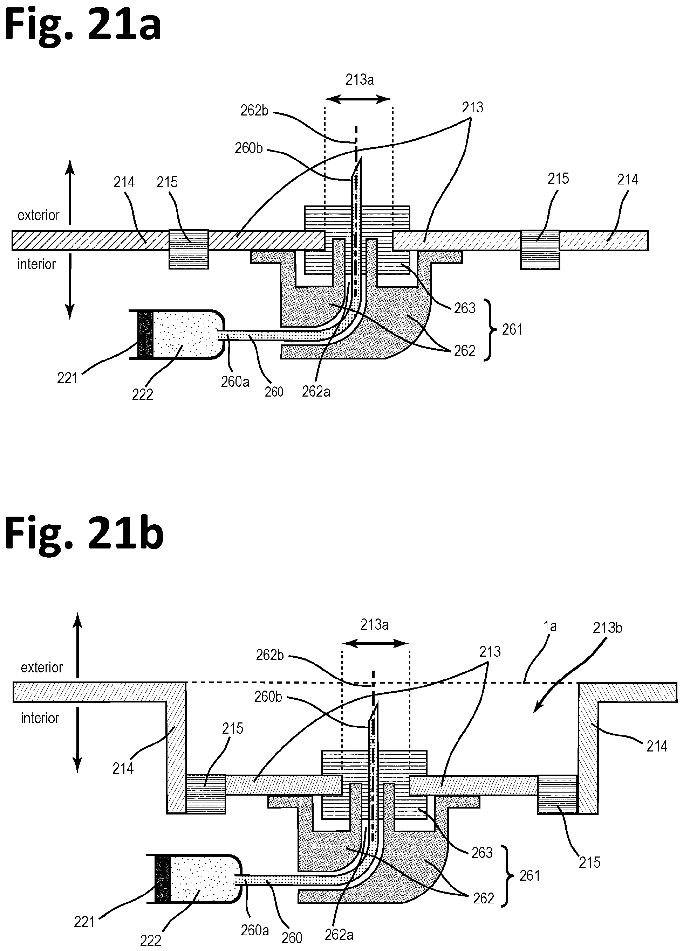

- Fig. 21a,b: refer to a schematic view of a patch pump with an exit port;
Fig. 21a depicts a patch pump with an exit port in a flat area of the housing
Fig. 21b depicts a patch pump with an exit port in a housing with a recess at the exit port

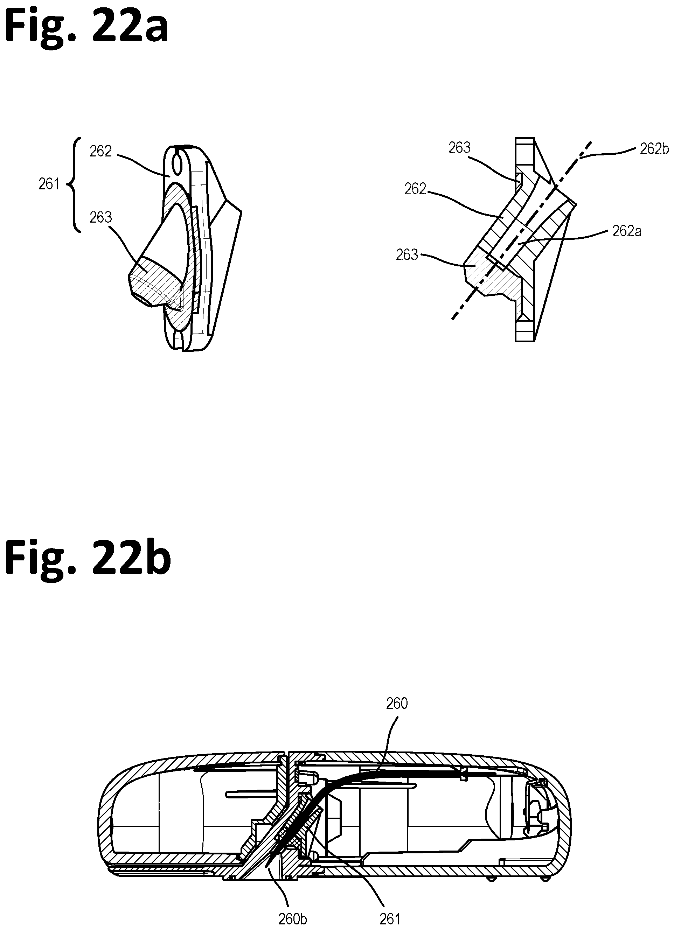

- Fig. 22a,b: refer to the exit port assembly of a first preferred embodiment of an exit port;
Fig. 22a depicts a preferred embodiment of the exit port assembly in perspective (left) and cut across (right);
Fig. 22b depicts a cut across an embodiment of a patch pump with the exit port assembly of figure 22a, fully assembled;

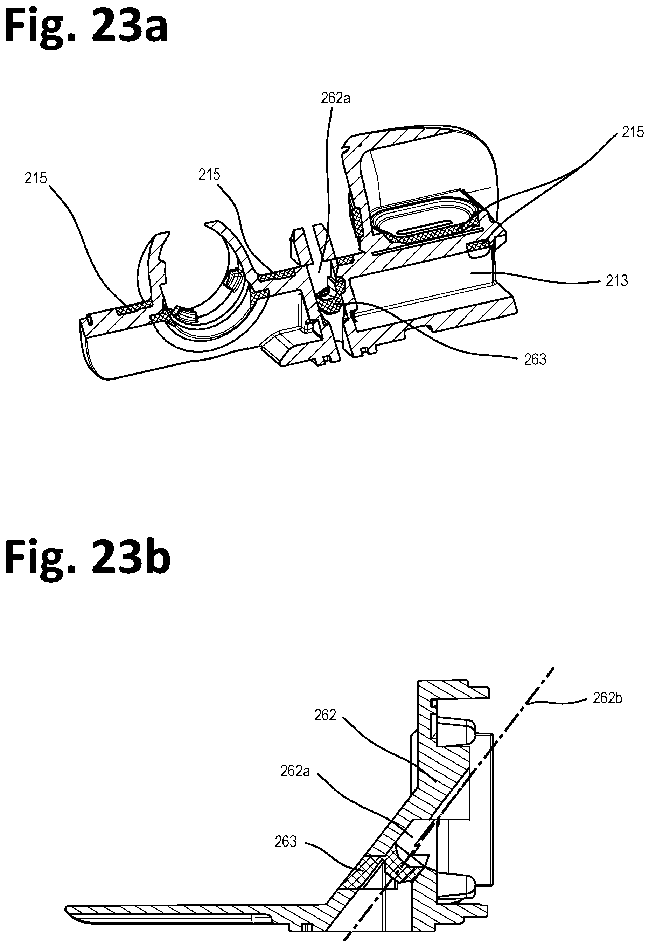

- Fig. 23a-c: refer to the exit port assembly of a second preferred embodiment of an exit port;
Fig. 23a depicts a preferred embodiment of the exit port assembly in perspective view, cut through the exit port channel;
Fig. 23b depicts a preferred embodiment of the exit port assembly cut across (right);

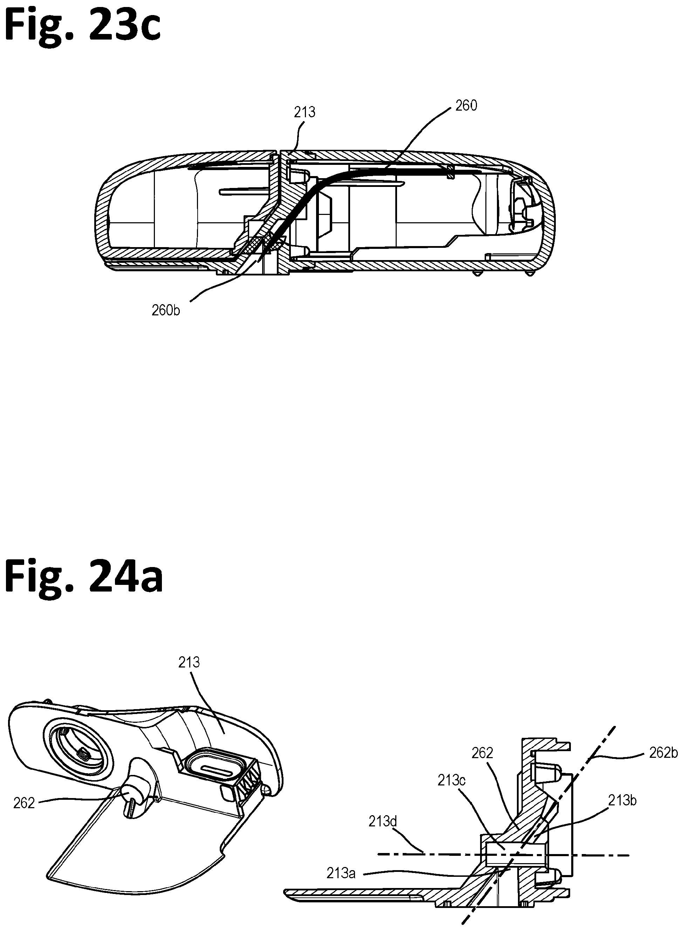

Fig. 23c depicts a cut across an embodiment of a patch pump with the exit port assembly of figure 23a, fully assembled;
- Fig. 24a-d: refer to the exit port assembly of a third preferred embodiment of an exit port;
Fig. 24a shows a housing component with integrated rigid exit port seal holder in a perspective view from outside (left) and a cut across the exit port (right), before inserting the soft exit port sealing;

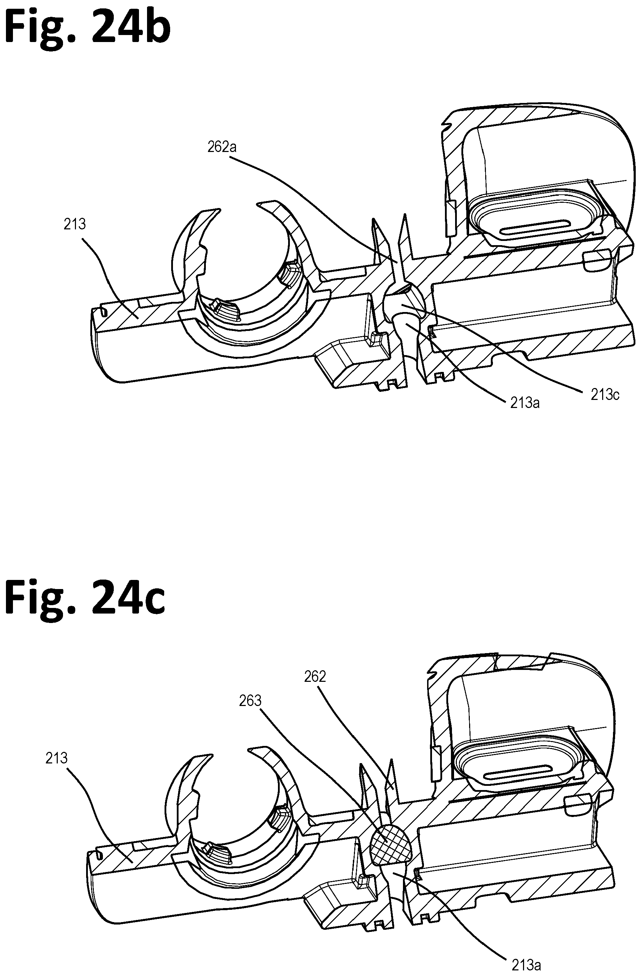

Fig. 24b depicts a cut through the exit port channel seen from inside the housing to further illustrate the exit port sealing plug cavity before assembly;
Fig. 24c depicts a cut through the exit port channel seen from inside the housing with the soft exit port sealing mounted into the exit port sealing plug cavity;

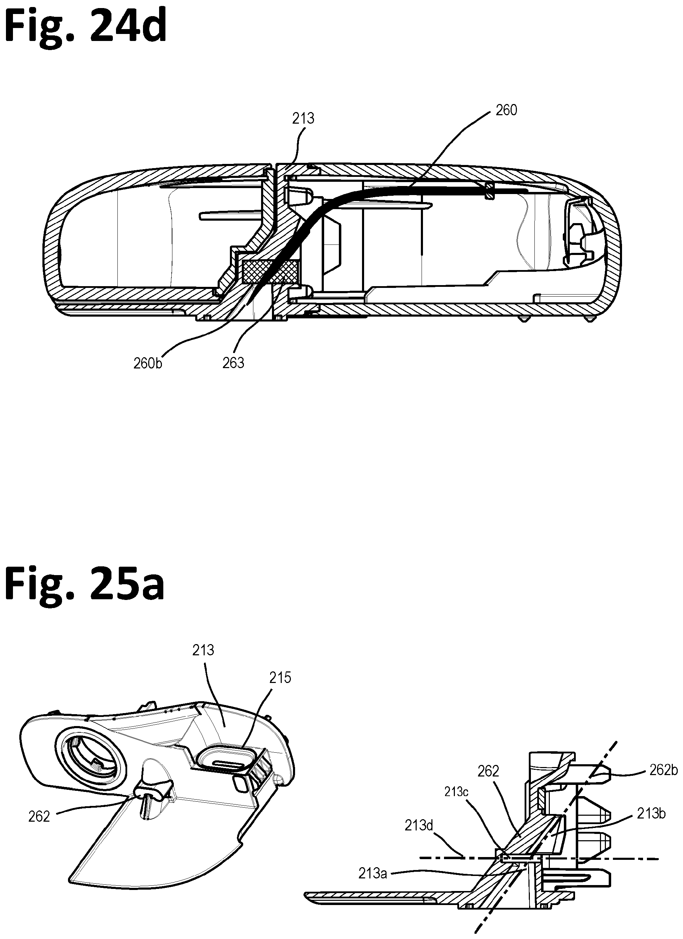

Fig. 24d depicts a cut across the exit port after piercing the sealing by the needle assembly;
- Fig. 25a-e: refer to the exit port assembly of a fourth preferred embodiment of an exit port;
Fig. 25a shows a housing component with integrated rigid exit port seal holder in a perspective view from outside (left) and a cut across the exit port (right), before inserting the soft exit port sealing;

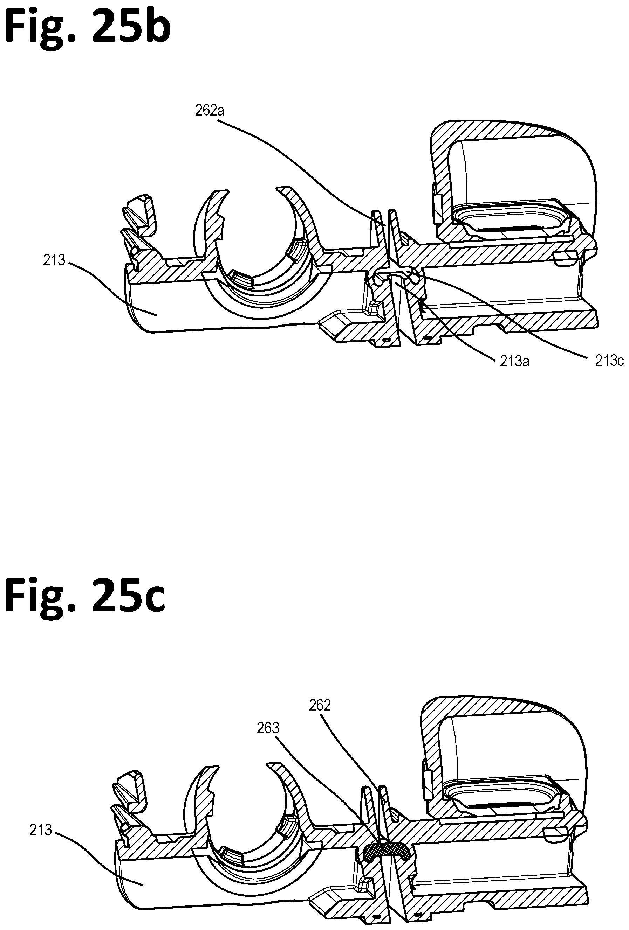

Fig. 25b depicts a cut through the exit port channel seen from inside the housing to further illustrate the exit port sealing plug cavity before assembly;
Fig. 25c depicts a cut through the exit port channel seen from inside the housing with the soft exit port sealing mounted into the exit port sealing plug cavity;

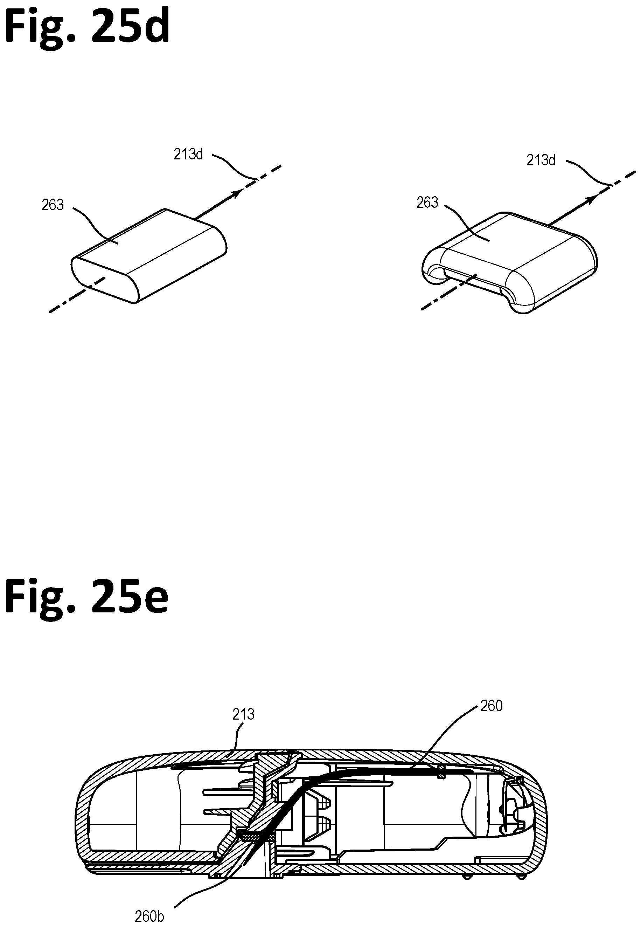

Fig. 25d depicts a soft exit port sealing, before (left) and after (right) insertion into the exit port sealing plug cavity of figure 25b
Fig. 25e depicts a cut across the exit port after piercing the sealing by the needle assembly;

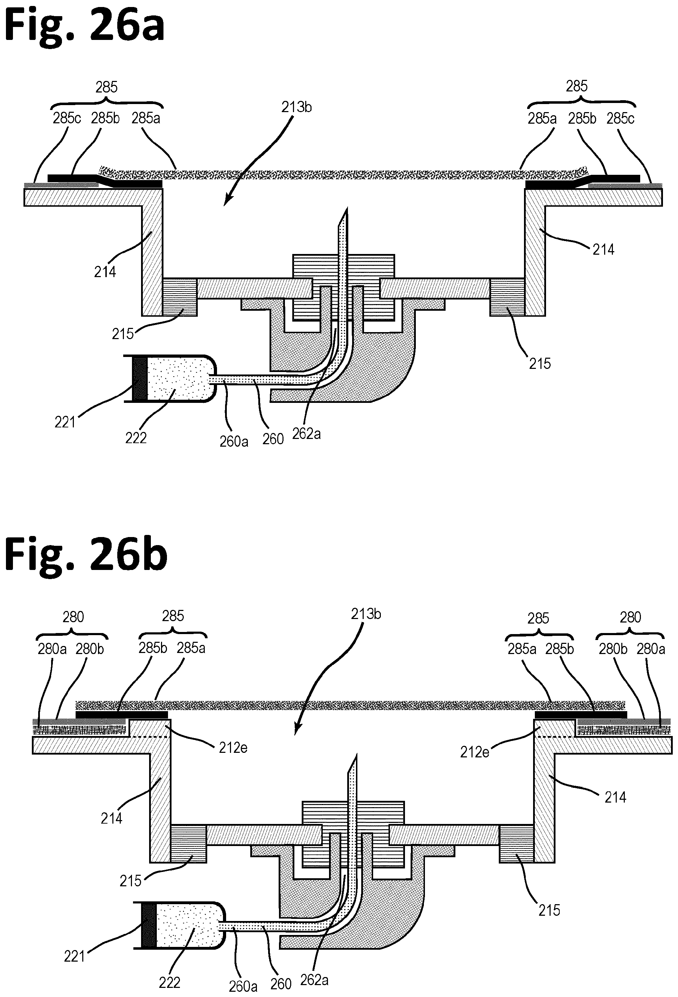

- Fig. 26a-c: refer to a an exit port with an exit port chamber and an exit port lid;
Fig. 26a depicts an exit port lid closing the exit port chamber;
Fig. 26b depicts an exit port lid in an embodiment with a membrane carrying structure

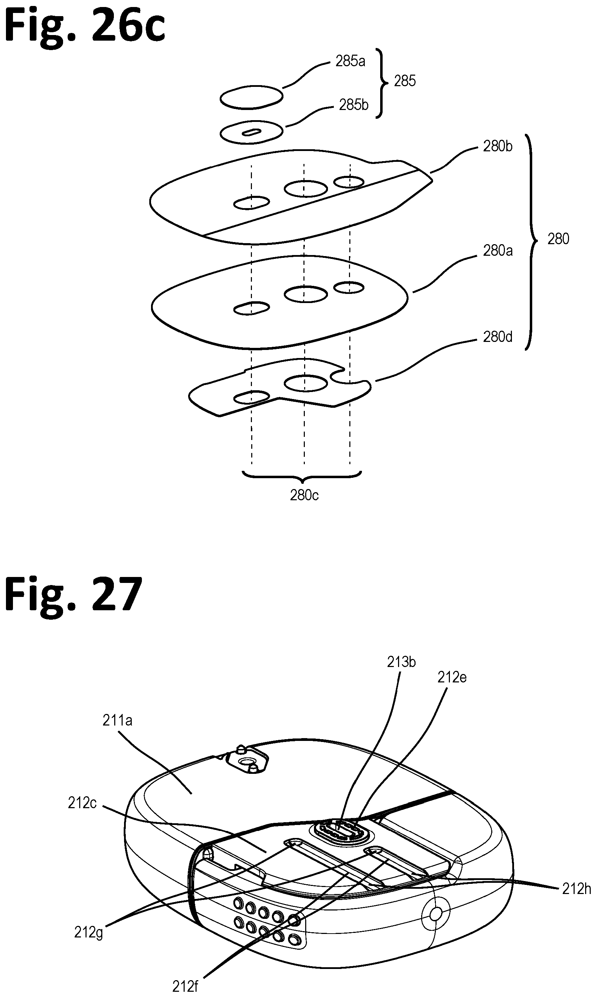

Fig. 26c depicts an exploded view of an adhesive assembly combined with an exit port lid;
- Fig. 27: depicts ventilation grooves at the bottom of a patch pump;

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

As outlined in the introductory paragraphs the present invention has a number of aspects, all contributing to an overall optimum of an improved drug delivery device which is accurate, reliable and easy to use while still suitable for conducting complex therapies at as low cost as possible. While most preferred embodiments are explained using the example of a wearable patch pump as shown in figures 1 and 2a,b, it is evident that the same aspects of the current invention may also be used to improve any other kind of drug delivery devices, wherever a comparable feature is present. Cost effectiveness is particularly realised by the introduction of new and improved manufacturing and/or assembly methods for the drug delivery device as described under the aspects to follow.

Figure 1 depicts a perspective view of a preferred embodiment of a drug delivery device according to the present invention. The drug delivery device is realised as a patch pump 1. The patch pump 1 comprises a reusable pump unit 100 and a disposable reservoir unit 200. The reservoir unit 200 comprises a reservoir to store the medicament and a needle assembly with a fluid path to bring the drug from the reservoir into the body of the patient. At the bottom of the reservoir unit 200 an adhesive patch assembly 280 is included to attach the patch pump 1 to the body of the patient. The pump unit 100 is releasably and sealingly connected to the reservoir unit 200 by a bayonet connection. Figure 1 (left) shows the complete patch pump 1 with both units connected and seen from a position above the pump. In the context of the present invention, "above" or "top" refers to the side of the pump which is facing away from the patient's body when the pump is attached to the patient for drug delivery. Consequently, "bottom" or "base" refers to the side of the pump facing towards the patient's body during drug delivery. In figure 1 (left), the arrow 114 indicates the direction towards the opening side of the patch pump 1, where the pump unit 100 is lifted off the bottom of the reservoir unit 200. Figure 1 (right) shows the same pump turned around for a bottom view of the patch pump 1, with the adhesive patch assembly 280.

As illustrated in figure 2a, the pump unit 100 comprises a drive mechanism for driving the plunger rod 122, an encoder to supervise the movement of the drive mechanism, a rechargeable battery and a programmable electronic system control circuitry configured to control the set-up, drug delivery and supervision of the pump. The battery can be recharged by a further battery in the disposable reservoir unit 200 while the drive mechanism is connected to the reservoir unit 200.

The drive mechanism 120 acts mechanically from a threaded rod 125 via plunger rod 122 on a plunger 221 in the reservoir 222 to dispense the medical substance out of the reservoir 222. A needle assembly 260 inside the reservoir unit provides the fluid connection from the reservoir 222 to the exterior of the pump for application to the patient. To ensure safe handling, the patch pump is manufactured, shipped, stored and prepared for use with the needle assembly 260 completely inside the enveloping shape of the pump 1. The enveloping shape is an imaginary surface enveloping the housing of the pump 1 while smoothly bridging all gaps and recesses, should any be present, to a closed shell. It is the shape of the pump 1 as perceived by the user from a distance and relevant when it comes to aspects of use like handling or wearability. Preparation of the patch pump in this embodiment includes filling the reservoir inside the reservoir unit 200 from the exterior using a transfer syringe and attaching the pump to the body of the patient by means of the adhesive patch assembly 280. An inserter assembly is included in the reservoir unit 200 and configured to bring an output portion of the needle assembly 260, in particular the open distal end of the needle assembly 260, out of the enveloping shape of the pump and into the body of the patient once the pump is ready to start drug delivery. In a preferred embodiment of a patch pump the needle assembly 260 includes a rigid cannula and a soft cannula, and the inserter is configured to insert the distal end of the soft cannula into the body of the patient using the rigid cannula, which will subsequently be retracted for drug delivery.

A rechargeable battery provides the power for the drive mechanism and for a system control circuitry. The latter controls the drive mechanism and may exchange data with an external controlling device, for example, via wireless data connection. Furthermore, the rechargeable battery may provide power for the inserter assembly in the reservoir unit 200.

Figure 2b shows the semi-disposable patch pump from figure 1 with the pump unit 100 and the reservoir unit 200 detached and separated, in a view from above, with the adhesive patch assembly 280 at the bottom towards the body of the patient, and the bayonet connection 212a in a disengaged state.

A first set of preferred embodiments illustrates the first aspect of the present invention. They are based on the wearable, semi-disposable patch pump shown in figures 1 and 2a,b as described before. They are further detailed in the following paragraphs.

The general aim of the invention is to provide a semi-disposable patch pump, optimised for ease of use, cost effectiveness and minimum waste. As evident from the general description of the embodiment of figures 1 and 2a,b, the patient has only two components to assemble: the reusable pump unit 100 with all the components intended for multiple or continuous use, and the disposable reservoir unit 200 with all the components intended for single use. Figure 2b shows a preferred embodiment of the semi-disposable patch pump according to the present invention with the pump unit 100 and the reservoir unit 200 ready for application. The reservoir unit 200 is shown from outside with the reservoir unit housing 211 mounted on the adhesive patch assembly 280, and the mechanical interface to the pump unit, in particular the bayonet connection 212a shown on both units, the rotation axis 212b of the bayonet connection shown on both units, and the locking structure 212d integrated in the reservoir unit housing 211. The locking mechanism for the bayonet connection includes the locking structure 212d on the reservoir unit and a flexible locking spring 113a on the pump unit (see figure 3b). A base plate 211a is integrated in the reservoir unit housing 211 and forms the bottom of the housing where the housing is attached to the adhesive patch assembly 280. Depending on the embodiment the base plate may be one unitary component or a combination of components permanently connected together and attached to the adhesive patch assembly 280. The design of the bayonet connection includes the definition of an opening side 114 of the pump, which refers to the general direction or outer face where the pump unit is lifted off the base plate to open the connection and remove the pump unit. In figure 2b the base plate is shown with a base plate extension 212c on the opening side 114. As the pump unit 100 is rotated around the rotation axis 212b of the bayonet, it is evident that the base plate extension 212c may only cover an area on the opening side of the pump starting from the rotation axis of the bayonet connection because it would otherwise inhibit connecting the pump unit to the reservoir unit.

A preferred embodiment of the reservoir unit is illustrated in figures 16 and 18, with selected parts removed to show the inner components. In particular, the reservoir 222 is shown with the plunger 221 and the reservoir outlet 222c leading to the needle assembly 260 mounted on an inserter assembly 250 which provides an auto-inserting mechanism. In this embodiment, the reservoir axis 222e along the axial centre of the reservoir 222 and the plunger 221 is arranged to coincide with the rotation axis 212b of the bayonet connection (figure 2b) and substantially orthogonal to the wall of the reservoir unit housing facing the pump unit in a fully assembled state.

A preferred embodiment of the pump unit is illustrated in figure 2a,b and figure 17, with selected parts removed to show the inner components. In particular, the drive mechanism 120 is shown with the driving means realised as a threaded rod 125 mounted in and in cooperation with a plunger rod 122, with the rechargeable battery 150 and the system control circuitry 140. The threaded rod 125 and the plunger rod 122 are arranged in substantially the same direction as the rotation axis 212b of the bayonet connection and substantially orthogonal to the wall of the pump unit housing facing the reservoir unit in a fully assembled state.

To apply the patch pump 1 and start drug delivery the patient takes the reservoir unit 200 out of the packaging. If the reservoir is not pre-filled with drug the patient fills the drug into the reservoir 222. The pump unit 100 will typically already be activated from prior use. If not, the patient may need to perform additional steps such as unpacking, charging or programming to prepare the pump unit. External devices and/or system components may be used, such as a remote control unit with a wireless connection and a software to interact with the user and set-up the communication with the pump unit. To attach the pump unit 100 to the reservoir unit 200, the user brings the bayonet 212a connector on the pump unit 100 in contact with the bayonet connector 212a on the reservoir unit 200 at an open angle, along the bayonet rotation axis 212b. Depending on the design of the bayonet construction, the open angle is typically in the range of 5 degrees to 180 degrees, preferably 15 degrees to 45 degrees. The user pushes the two components along the rotation axis of the bayonet connection 212a together and folds the pump unit 100 down onto the plane of the base plate 211a. In the example of the preferred embodiment the plunger rod cap 123 at the tip of the plunger rod 122 in the pump unit is connected to the opening behind the plunger 221 in the reservoir unit. With the bayonet components in place, the pump unit 100 is folded down onto the plane of the base plate 211a to close the connection. Figure 3a shows this step with the pump unit at the connecting angle of 30 degrees. The user makes sure the bayonet connection is properly locked by pressing down the pump unit 100 onto the base plate 211a. By doing so, the flexible latch spring 113a of the reusable pump unit engages with the locking structure 212d integrated in the housing of the disposable reservoir unit 200, and locks the bayonet connection in closed position. This state is illustrated in Figure 3b. It is evident that the bayonet connection of the present invention may also include any other locking mechanism such as a magnetic lock, velcro, latch or adhesive lock, provided the lock can be released without introducing an additional unlocking step.

In figure 4, the patch pump 1 has been applied to the body of the patient 300 and is ready for drug delivery. The enveloping surface or outer shape or of the patch pump 1 is substantially edge-free and suitable for wearing with minimal risk of brushing the device off when sliding over an edge such as a door frame.

After successful delivery of the drug inside the reservoir, the patient removes the pump from his or her body. One single mechanical handling step is required to remove the pump unit from the used reservoir unit and be ready for the new one. The bayonet connection is opened by simply lifting the pump unit 100 off the plane of the base plate 211a at the opening side 114 of the pump. The same movement will unlock the locking mechanism and open the bayonet. To further ease this handling step, a cut-out 211m may be made on the base plate 211a or the base plate extension 212c on the opening side 114 of the pump 1. The cut-out 211m reduces the size of the base plate 211a at the location where the finger of the patient grips the pump unit 100 for unlocking and detaching from the reservoir unit 100. The cut-out may have arbitrary shape, preferably elongate, along the edge of the base plate 211a, about as long as the width of a finger and wide enough to generate a haptic effect. Accordingly, the size of the cut-out 211m along the base plate 211a and/or the base plate extension 212c will typically be 5 mm to 30 mm in length along the edge and 1 mm to 10 mm in width away from the original edge of the base plate 211a and/or base plate extension 212c. In figure 3a, an example of the cut-out is shown at the edge of the base plate extension 211a. When folded up to the open angle of the bayonet connection, the pump unit can be separated from the reservoir unit and attached to a new reservoir unit to continue with the therapy.

Another set of preferred embodiments illustrates the second aspect of the present invention. They are based on the wearable, semi-disposable patch pump shown in figures 1 and 2a,b as described before. They are further detailed in the following paragraphs.

Figures 5, 6a and 7 (bottom-up) show the reservoir unit 200, with selected parts removed to allow a view inside the reservoir unit 200. The reservoir unit 200 comprises a reservoir unit housing 211, a reservoir 222 with the medical substance, a hybrid assembly 240 according to the invention, the button cell battery 244 and an adhesive patch (not shown) for connecting the patch pump 1 to the skin of the user.

Figures 6a, 6b and 7 depict the hybrid assembly 240, which is arranged inside the reservoir unit housing 211 of the reservoir unit 200 as shown in figure 5. The hybrid assembly 240 comprises parts of the inserter assembly 250, in particular a cannula moving assembly 251. Figure 6a depicts the hybrid assembly 240 with the cannula moving assembly 251 in a biased initial position. The inserter assembly 250 brings the cannula moving assembly 251 from the biased initial position into an extended position for drug delivery. This movement may be driven for example by a system of elastic elements like the insertion spring 252 (figure 7) built into the inserter mechanism, with at least one of the elastic elements being pre-loaded during manufacturing of the patch pump. Figure 6b depicts the hybrid assembly with the cannula moving assembly 251 in an extended position for drug delivery. Figure 7 shows a bottom view of the hybrid assembly 240.

As shown in figure 6a the hybrid assembly 240 comprises additionally a printed circuit board PCB-RU 243, the button cell battery 244 and a base frame 241 for supporting the hybrid assembly 240, the PCB-RU 243 and the battery 244.

In the following the structural features of the base frame 241 are described in detail. Subsequently, the function of the hybrid assembly 240 is described.

Figure 8a depicts the base frame 241 alone. The base frame 241 comprises a connector structure 270 with four connector members 271a-271d and a non-conductive body 290 made of plastic, which is injection-moulded around the connector structure 270.

In a first production step the connector members 271a-271d are stamped out of a metal sheet. At this stage, the connector members 271a-271d are physically connected to each other by bridges, which are temporary connecting metal elements. In a second step the connector members 271a-271d of the stamped out metal sheet are bent to form electrical contact areas and arms. In a third step the connector members 271a-271d are overmoulded by non-conductive plastic to form the non-conductive body 290 around the inlaid metal structure. In a fourth step the bridges between the connector members 271a-271d are eliminated by stamping to galvanically separate the connector members 271a-271d from each other. The connector members 271a-271d are made of electroconductive metal. The injection-moulded non-conductive body 290 is made of a non-electroconductive plastic.

Each of the four connector members 271a-271d comprises a contacting arm 272 (figure 8c). Each contacting arm comprises bendings. A first free end of the contacting arm 272 forms a first electrical contact area 272a as shown in figure 8c.

The first 271a, the second 271b and the third 271c connector member each comprise a second end of the contacting arm 272 with a second electrical contact area 272b adapted for electrically contacting the PCB-RU 243 supported by the non-conductive body 290 (see figure 8a, 6ab).

The first connector member 271a additionally comprises a switching arm 273 and a first battery contact arm 274 shown in figure 8c. The switching arm 273 is integrally formed in the first connector member 271a and is resiliently held relative to the rest of the connector member. The switching arm 273 comprises on its free end a contact surface adapted to establish an electrical contact to an electrical contact area of the pump unit printed circuit board PCB-PU 141 (see figure 17) when the pump unit 100 is connected to the reservoir unit 200. The first battery contact arm 274 comprises on its free end a first battery contact area 274a adapted to be connected to the positive pole of the button cell battery 244.

The fourth connector member 271d comprises only the first electrical contact area 272a and a second battery contact arm 275, which comprises at its free end a second battery contact area 275a adapted to be connected to the negative pole of the button cell battery 244.

With reference to figure 8a-8c the non-conductive body 290 is described in detail. The non-conductive body 290 comprises a first opening 291 extending through the non-conductive body, through which opening the first electrical contact areas 272a of the contacting arms 272 can be contacted from outside the base frame 241. Through a second opening 292 in the middle of the non-conductive body 290 the second electrical contact areas 272b can be electrically connected to the reservoir unit printed circuit board (PCB-RU) 243. The switching arm 273 protrudes also through the second opening 292. On a bottom side of the non-conductive body 290 the first and second battery contact arms 274, 275 reach out of the non-conductive body 290 such that the first and second battery contact arms 274, 275 can contact the positive pole and the negative pole of the battery 244, respectively.

As depicted in figure 8a the non-conductive body 290 integrally forms a linear guiding for the cannula moving assembly 251, which comprises a soft cannula holder 253 and a rigid cannula holder 254 (see figures 6a, 6b, 7). As depicted in figure 8a-c the linear guiding comprises au upper guiding rail 295 and a lower guiding rail 296. The upper guiding rail 295 is adapted to guide the soft and rigid cannula holder 253, 254 such that the holders are shiftable between the retracted position and an extended position, which is at a second end of the guiding rails 295, 296. The lower guiding rail 296 is adapted to guide a retraction control part for retracting the rigid cannula holder 254 such that the control part is linearly shiftable along the lower guiding rail 296. At a first end of the guiding rails 295, 296 is an end stop surface 297 arranged, which restricts a linear movement of the soft and rigid cannula holder 253, 254 and defines an initial or retracted position of the holders 253, 254.

On an upper side, four fixation pins 293 are integrated into the non-conductive body 290. During assembly the reservoir unit printed circuit board (RU- PCB) 243 is mounted on the four fixation pins 293 and fixated by heat staking.

On a bottom side of the base frame 241 the non-conductive body 290 forms a battery opening 245 (shown in figure 7) adapted for accommodating the button cell battery 244. A retaining element 246 for holding the battery 244 is arranged next to the battery opening 245. The retaining element 246 as depicted in figure 7 is integrally formed in the non-conductive body 290. When a battery is inserted into the battery opening 245 the retaining element 246 is resiliently deflected to facilitate the insertion of the battery. If the insertion is completed and the battery is placed into the battery opening 245 the retaining element 246 resiliently moves back and thus holds the battery 244 in the battery opening 245.

Figure 8 shows the base frame 241 as a complete unitary component (figure 8a), in a view of the non-conductive body 290 without the inlayed conductive structure (figure 8b) and in a view of the conductive structure as present after manufacturing has been completed (figure 8c). As best visible in figure 8b, the non-conductive body 290 further integrally forms a cone-shaped bearing pin 294 on the upper side of the non-conductive body 290. The bearing pin 294 is adapted to pivotally support an insertion trigger 256.

In the final assembled state the complete hybrid assembly 240 is placed inside the reservoir unit housing 211 of the reservoir unit 200 as shown in figure 5. If the pump unit 100 of the patch pump 1 is connected to the reservoir unit 200 by the bayonet connection 212a connecting pins 142 (see figure 17) of the pump unit 100 are pressed onto the first electrical contact area 272a of each of the contacting arms 272. Thereby, an electrical connection is established between the battery 244 of the reservoir unit 200 and the system control circuitry 140 in the pump unit 100 and between the reservoir unit printed circuit board PCB-RU 243 and the system control circuitry 140 in the pump unit 100.

Through the electrical connection between the pump unit 100 and the reservoir unit 200 a rechargeable battery 150 in the pump unit 100 can be charged by the button cell battery 244 in the reservoir unit 200. By means of the electrical connection between the PCB-RU 243 and the PCB-PU 141 in the pump unit 100 the system control circuitry 140 can control a heating element of a heater assembly on the PCB-RU 243. In the same way, the system control circuitry may control other elements on the PCB-RU 243 and/or obtain and process information about the status of the reservoir unit such as the position of the needle assembly.

If the control circuitry 140 of the pump unit 100 sends a release command to the PCB-RU 243 in the reservoir unit a heating element of the heater assembly is activated. The heating element may then heat up a fuse to cause the fuse to melt, to extend or to break. This may trigger the release of the biased insertion trigger 256, which is pivotally supported by the bearing pin 294 (see figures 6a and 6b). After the biased insertion trigger 256 is released it can rotate away from the soft and rigid cannula holder 253, 254 and thus release them. After the release of the holders 253, 254 they move to the extended position, driven by a spring 252 guided by the guiding rails 295, 296 and thereby insert a soft and hard cannula into the skin of the user. The insertion mechanism is described in detail in the European Patent Application EP 18199475.7 which is hereby incorporated in its entirety.

Another set of preferred embodiments illustrates a third aspect of the present invention. They are based on the wearable, semi-disposable patch pump shown in figures 1 and 2a,b as described before. They are further detailed in the following paragraphs.

Figure 9a shows a 3-dimensional overview of a reservoir unit 200 comprising a reservoir or cartridge 222 and a fill port assembly 230, turned bottom up where in this example the fill port is located. The reservoir unit 200 comprises a housing 211 formed by a bottom wall 211g, a side wall 211h and a top wall 211i. The walls of the housing define an interior space having an opening for, amongst others, receiving the reservoir 222. The fill port assembly 230 is preferably located in the bottom wall 211g but may also be located in the other wall sections. The fill port assembly 230 is used for filling an empty reservoir 222 which is closed by a plunger 221, see figure 9b. Details of the fill port assembly 230, the housing and the reservoir 222 are presented in cross sectional views for:
- an assembled and fixated fill port assembly 230 in figure 9b,
- an assembled but not yet fixated fill port assembly 230 in figure 10,
- a partially assembled fill port assembly without an insert 235 in figure 11, and
- the assembly of the housing and the reservoir in figure 12.

As best visible in the exploded view of figure 16 and in the cross-section of figure 12, the housing 211, preferably the bottom wall 211g comprises a passage 211c adapted to receive the fill port assembly 230 formed by an insert 235 and fill port sealing 231. The passage 211c in the wall of the housing is surrounded by recesses or recessed sections (211d, 211f) and the passage 211c is aligned with the inlet 222a of the reservoir 222 to form an opening for receiving the fill port assembly. The housing 211, preferably the bottom wall of the housing 211g comprises at least one fixation pin 211e preferably made from the same thermoplastic polymer as the housing.

To properly position and fixate the reservoir inside the housing of the drug delivery device, a stabilising protrusion 222b is integrated in the reservoir. When the reservoir is inserted into the housing of the drug delivery device the stabilising protrusion 222b slides into a recess in the inner wall of the housing, in figure 12 shown as stabilising recess 211k. With the stabilising protrusion positioned in the stabilising recess 211k, the reservoir is properly positioned and laterally fixated to allow lateral pressure for mounting the fill port assembly. Further, the interaction between the stabilising protrusion and the stabilising recess may be designed as a snap fit connector (not shown in figures 12 and 16).

Once the reservoir 222 has been inserted and guided into the housing 211, preferably until the reservoir abuts a stop in the housing, the inlet 222a of the reservoir is aligned with the passage 211c in the housing. The stop in the housing may be formed by a protrusion or protruding structure on the housing abutting a portion of the reservoir.

To establish a fluid path from the exterior to the reservoir inlet 222a and ensure fluid-tightness at a specified pressure such as the filling pressure, the fill port assembly comprises at least one sealing. In the embodiment shown in figure 16, a fill port sealing 231 is inserted into the passage 211c of the housing along a longitudinal axis formed by the inlet 222a of the reservoir. The longitudinal axis of the inlet 222a is preferably perpendicular to the longitudinal axis 222e of the reservoir. The fill port sealing 231 comprises a cylindrical section 231f connecting a flange 231a to a pierceable septum 231c (figure 14). The axis of the cylindrical section 231f is aligned with the axis of the inlet 222a of the reservoir when the fill port sealing has been inserted into the passage 211c. The flange 231a has a lateral dimension that tightly fits into the second recessed section 211d of the housing 211 whereas the pierceable septum 231c tightly fits into the inlet 222a of the reservoir. The flange 231a is disc shaped having a cone shaped opening 231g for accessing a bore 231b ending in the pierceable septum 231c. The flange 231a fits in a disc shaped second recessed section 211d in the housing. The cylindrical section 231f forms the bore 231b that is adapted to receive a sleeve 235c of the insert 235 (see below). The entrance of the fill port sealing 231 starting from the flange is cone shaped and is adapted to receive a cone shaped opening 235a of the insert 235 (figure 13). The longitudinal axis of the cone of the insert coincides with the longitudinal axis of the inlet of the reservoir. The fill port sealing 231 is made, in this example, both from an elastomer and a thermoplastic polymer. Alternatively only an elastomer is used. The flange 231a and the outside surface of the cylindrical section 231f are made from the elastomer such that the flange can form a sealing towards the housing and the distal end of the cylindrical section 231f can form a sealing with the inlet 222a of the reservoir 222. The cone section of the fill port sealing 231 is made from the thermoplastic polymer to provide mechanical strength to the fill port sealing 231. The fill port sealing 231 is shown as a unitary component in figure 14. The fill port sealing is inserted into the fill port sealing cavity 222g of the reservoir 222 shown in figure 15. The fill port sealing 231 is preferably made using 2-component injection moulding with a rigid thermoplastic 231d like PBT and a soft elastomer 231e like polysiloxane liquid silicone (LSR) rubber.

A cross section of the fill port sealing 231 that has been inserted into the passage 211c in the bottom wall 211g of the housing is presented in figure 11. The distal surface of the flange 231a contacts the proximal surface of the second recessed section 211d of the housing (see figure 12). Optionally, the housing comprises a protruding rim 211j contacting the flange 231a of the fill port sealing 231. The distal end of the cylindrical section 231f of the fill port sealing 231 (figure 14) has an outer dimension that is above the inner dimension of the inlet 222a of the reservoir (figure 12) such that the distal end is radially compressed as it enters the reservoir thus establishing a second sealing 239 between the fill port sealing and the inlet 222a of the reservoir (figure 9b). The second sealing 239 is radially oriented perpendicular to the axis of the inlet of the reservoir. Optionally, the distal end of the cylindrical section and/or the pierceable septum 231c forms an axial sealing with a surface of the inlet of the reservoir.

The exploded view of figure 16 shows how the fill port sealing 231 and the insert 235 are inserted into the passage 211c of the housing. Figure 10 shows a cross section of the fill port assembly in this state, before fixation. The insert 235 is made from a metal (stainless Cr-Ni steel, for example AISI 305) and comprises a cone shaped opening 235a connecting a base 235b to a sleeve 235c (see also figure 13). The cone shaped opening 235a fits into the cone of the fill port sealing 231 formed by the thermoplastic polymer 231d. The sleeve 235c fits into the bore 231b of the sealing for guiding a needle towards the pierceable septum 231c. The base 235b of the insert comprises cut-outs or openings 235d which are adapted to be received by the at least one fixation pin 211e extending from the housing and the outer dimensions of the base as such fit into the first recessed section 211f of the housing (figure 10). The insert 235 is fixated to the housing by heat staking of the fixation pin 211e, thus heating and deforming the fixation pin 211e (figure 9b). During fixation of the insert 235, the flange 231a of the fill port sealing 231 is axially compressed between the base 235b of the insert and the bottom wall 211g of the housing. Optionally, the flange is locally compressed by the protruding rim 211j present in the second recessed section 211d in the housing (figure 11). Due to the compression of the flange, a first sealing 238 is formed that is axially displaced from the second sealing 239. The first and second sealings 238, 239 are established by the fill port sealing and bridge the gap between the inlet of the reservoir 222 and the housing 211 and prevent leakage from the reservoir into the housing and into the exterior (figure 9b). Moreover, the first and second sealings 238, 239 prevent contamination from the exterior into the drug delivery device or into the reservoir

As the insert is fixated to the housing, also lateral movement of the reservoir 222 with respect to the housing may be limited or even act as an impact absorber for the reservoir. The inlet 222a of the reservoir is coupled to the housing via the fill port sealing 231 and the insert 235 and the elastomeric material of the fill port sealing 231 may act as a cushion or shock absorber between the rigid reservoir and rigid housing.

A method for assembling the drug delivery device or a part of the drug delivery device is shown in figure 16. The reservoir 222 is inserted into the opening of the housing along the longitudinal axis 222e of the reservoir. The plunger 221 may already be present in the reservoir (figure 9b) or is inserted into the reservoir after the reservoir is positioned inside the housing 211. Preferably, the wall of the reservoir is reinforced with a number of reinforcing ribs 222f also adapted to facilitate the orientation and guidance of the reservoir in the housing 211 during assembly. The housing of the drug delivery device may further have a stop surface which, when abutted by the reservoir, ensures that the inlet 222a of the reservoir is aligned with the passage 211c of the housing 211. The inside surface of the housing may have longitudinal ridges for correctly guiding the reservoir to its final position. In a subsequent step, the fill port sealing 231 and the insert 235 are inserted into the passage 211c in the housing along an axis that is defined by the cone shaped opening 235a of the insert (or the cone shaped opening of the fill port sealing). The axis defined by the cone shaped opening 235a is preferably identical to the axis defined by the inlet 222a of the reservoir when the reservoir is in its final position. The distal end of the fill port sealing enters the inlet 222a of the reservoir to establish the second sealing 239 during insertion of the fill port sealing. During fixation of the insert, preferably by heat staking of the fixation pins 211e, the first sealing 238 is established. Before, during or after insertion of the reservoir, the hybrid assembly 240 is inserted into the housing as well.

Another set of preferred embodiments illustrates a 4^{th} aspect of the present invention. They are based on the wearable, semi-disposable patch pump shown in figures 1 and 2a,b as described before. They are further detailed in the following paragraphs.

The reservoir outlet sealing is improved by a design which is particularly cost-effective to manufacture. Such an advantageous design has already been described with the third aspect of the present invention, where the reservoir outlet sealing is combined with an improved fill port. Figure 18 shows an additional way to improve the reservoir outlet sealing. The reservoir unit of figure 1 is shown with part of the housing removed, followed by a more detailed cut across the outlet of the reservoir (figure 19). The reservoir 222 has a rigid structure forming a reservoir outlet 222c. In this example, the reservoir outlet has a substantially cylindrical reservoir outlet sealing cavity 222d. During manufacturing, the reservoir outlet sealing cavity 222d is closed by inserting a substantially cylindrical reservoir outlet sealing 223 made of soft, fluid-tight but pierceable material such as silicone or any kind of rubber, establishing a fluid-tight closure of the reservoir outlet sealing cavity 222d. In a separate manufacturing step the reservoir outlet sealing 223 is pierced by the input portion of the needle assembly. In the current example of an embodiment, the input portion of the needle assembly consists of the input portion of the rigid cannula 258. The result is a fluid-tight connection between the outlet of the reservoir and the needle assembly. Unlike with a membrane-like sealing, the substantially cylindrical shape of the reservoir outlet sealing and consequently of the reservoir outlet sealing cavity provides a considerably improved fluid-tightness, in this example up to a specified filling pressure of 6 to 8 bar. A large range of variations of this embodiment are possible within the scope of the current invention. The reservoir outlet sealing cavity and the reservoir outlet sealing may only by partially cylindrical or have a different shape altogether, while keeping the area of contact between the reservoir outlet sealing cavity and the reservoir outlet sealing large enough to provide the required fluid-tightness. Manufacturing of the reservoir outlet may vary by changing the sequence of manufacturing steps, for example by piercing the reservoir outlet sealing before or after inserting the reservoir outlet sealing into the reservoir outlet sealing cavity. More variations are generated by applying the improvements to the exit port sealing to the reservoir outlet. All features described under the 4^{th} aspect of the current invention, for example reducing the number of components by applying 2-shot injection moulding technology or improving manufacturability by introducing an exit port sealing plug cavity which intersects the outlet port opening, also lead to variations of the embodiments as described for the reservoir outlet.

Another set of preferred embodiments illustrates a 5^{th} aspect of the present invention. They are based on the wearable, semi-disposable patch pump shown in figures 1 and 2a,b as described before. They are further detailed in the following paragraphs.

In figure 18, the reservoir unit 200 of figure 1 is shown with part of the housing removed, in particular revealing the rigid cannula 258 and the soft cannula 259 which are the main components of the needle assembly in this embodiment. Figure 20 shows a cut across the needle assembly at the interface between the rigid cannula 258 and the soft cannula 259. The rigid cannula 258 has a substantially tubular shape and is configured to slide axially in a soft cannula lumen 259a of the soft cannula while maintaining a fluid-tight sealing connection at the proximal input end of the soft cannula 259. To achieve fluid-tightness up to the specified filling pressure of 6 to 8 bar, the soft cannula has a soft cannula input sealing portion 259b, where the material of the soft cannula 259 is deformed, thickened or otherwise altered to increase the pressing of the soft cannula on the surface of the rigid cannula. The cannula input sealing portion 259b of the soft cannula 259 may be formed during manufacturing of the needle assembly or a later manufacturing step of the pump by injection moulding, by applying heat and/or mechanical pressure from outside the needle assembly, or by other means to thicken and/or deform the soft cannula 259. While the most preferred location of the cannula input sealing portion 259b is right at the proximal input of the soft cannula 259, variations of this embodiment are well possible with the soft cannula input sealing portion 259b further towards the output end of the soft cannula 259. Further variations may have the soft cannula input sealing as a separate component from the soft cannula.

Another set of preferred embodiments illustrates a 6^{th} aspect of the present invention. They are based on the wearable, semi-disposable patch pump shown in figures 1 and 2a,b as described before. They are further detailed in the following paragraphs.

The exit port is improved by reducing the number of components at this interface by integrating one or more originally separate components into one unitary component. Figure 21a gives a schematic overview over the concept applied at the exit port of the drug delivery device. The housing of the pump is shown with a first housing component 213 around the exit port opening 213a and two other housing components 214 connected to the first housing component 213 via housing sealings 215. The combination of housing components, both of the pump unit and the reservoir unit, when equipped with a fill port and/or an exit port and/or other sealing elements suitable for the intended use and connected for said intended use, form a protective shell to protect the pump from mechanical damage, contamination or other form of environmental ingress. An exit port assembly 261 comprising a rigid exit port sealing holder 262 and a soft exit port sealing 263 is mounted at the exit port opening 213a of the housing. To support the manufacturing process of the patch pump, the rigid exit port sealing holder 262 not only holds the soft exit port sealing 263 in place for closing the exit port opening 213a, but also serves as a mechanical guide for the process of piercing the soft exit port sealing 263 with the output portion 260b of the needle assembly 260. The rigid exit port sealing holder 262 may have any shape suitable for that purpose. Examples of simple shapes include a straight tube, a bent tube, a cone, a U-shaped groove or a combination of these shapes. At the point of piercing, where the output portion 260b of the needle assembly 260 first comes into contact with the soft exit port sealing 263, the shape of the rigid exit port sealing holder is tubular, which defines an at least partially tubular exit port channel 262a with an axis 262b.

The rigid exit port sealing holder 262 is attached to one of the housing components, in figure 21a the first housing component 213. The soft exit port sealing 263 is mounted at the exit port opening 213a to fluid-tightly close the exit port opening, while at the same time establishing a fluid-tight sealing between the rigid exit port sealing holder 262 and the housing, in figure 21a the first housing component 213. This has the advantage that the connection between any housing component and rigid exit port sealing holder 262 does not need to be fluid-tight at all. The requirement for fluid-tightness of the exit port is fully ensured by the soft exit port sealing, which can be designed to withstand the pressure as specified, be it environmental pressure, occlusion pressure or filling pressure. The soft exit port sealing 263 is made of a soft and elastic material, such as an elastomer like silicone rubber or any other fluid-tight material which is pierceable by the output portion 260b of the needle assembly 260 and elastic enough to provide the surface pressure required for fluid-tight sealing at the specified fluid pressure. Other examples for such an elastomer may by an EPDM rubber, polydimethylsiloxane rubber PDMS, preferably in LSR form or an elastomeric polyurethane (PUR) or a thermoplastic elastomer (TPE). The soft exit port sealing 263 is attached to the rigid exit port sealing holder 262 during manufacturing to form a fluid-tight connnection, for example by2-shot injection molding, by press-fit or by any other means of fixating the sealing for the intended use. The process of manufacturing the patch pump of this embodiment includes the step of attaching the rigid exit port sealing holder to the housing, while thereby closing the exit port opening, and the step of piercing the soft exit port sealing by the output portion 260b of the needle assembly 260. As the output portion of the needle assembly is intended to be inserted into the body of the patient for drug delivery, said output portion 260b will typically have a rigid and pointed end, such as the tip of a sharpened needle or rigid cannula. The sequence of manufacturing steps is not relevant for the function of the exit port, hence the piercing of the exit port sealing 263 may take place before or after mounting the exit port assembly 261 into the housing of the drug delivery device. As the soft exit port sealing has two sealing functions, the step of attaching the rigid exit port sealing holder to the housing may be the same as the step of attaching the soft exit port sealing to the rigid exit port sealing holder.

The result is a patch pump which is easy to manufacture and has a unitary exit port sealing fulfilling three sealing functions in one: sealing the housing to the rigid exit port sealing holder, sealing the output portion of the needle assembly to the rigid exit port sealing holder, and fluid-tightly closing the exit port opening.

A further improvement of the exit port concept of the present invention is achieved by modifying the shape of the housing of the drug delivery device and by introducing a recess in the area of the exit port. This alternative is shown in the schematic overview of figure 21b using the same numerals for the same parts as in figure 21a. The recess in the housing creates an exit port chamber 213b and the exit port opening 213a now lies sufficiently far in the interior of the enveloping surface 1a of the pump to keep the output portion 260b of the needle assembly 260 completely in the interior. This arrangement has the advantage of protecting the output portion of the needle assembly from inadvertent physical contact with the environment, contamination or damage, while at the same time protecting the user and/or patient from inadvertent contact with the output portion of the needle assembly and hence reducing the risk of harm by pricking.

The exit port concept of the present invention leads to at least three main groups of further improved embodiments. In a first group, the exit port is improved by combining the components of the exit port assembly, the rigid exit port sealing holder and the soft exit port sealing to one unitary component . Figure 22a,b illustrates a preferred embodiment of this group. Figure 22a shows the exit port assembly 261 in a perspective view (left) and in a cut across (right). The exit port assembly 261 is manufactured as one unitary component, for example by 2-shot injection moulding, with the rigid exit port sealing holder 262 and the soft exit port sealing 263 injected in separate shots using different materials as defined by the design described before. Alternatively, the exit port assembly 261 may be pre-manufactured by attaching two components, for example by gluing, by applying press-fit or any other technology resulting in a fluid-tight connection between the two components. In figure 22a, the multiple sealing function of the soft exit port sealing is well visible: the sealing does not only cover the open distal end of the exit port channel 262a, but also comprises a sealing area surrounding the exit port channel to seal the interface to the housing and close the exit port opening in an assembled state. Figure 22b shows a patch pump in an embodiment using the exit port assembly of figure 22a, in a fully assembled state, including the needle assembly 260 with the output portion 260b.

In a second group of embodiments according to the 6^{th} aspect of the present invention, the exit port is improved by combining the exit port assembly with one of the components of the housing to one unitary component. Figure 23a-c illustrates one of many possible preferred embodiments of this group. Figure 23a shows the first housing component 213 with all soft components attached in a perspective view with a cut along the exit port channel axis. The first housing component 213 is manufactured as one unitary component, for example by 2-shot injection moulding with a soft material and a rigid material. The rigid exit port sealing holder 262 is an element of the first housing component 213, made of rigid material, and manufactured for example in a first shot of injection moulding. The soft exit port sealing 263 is for example injected in a second shot of injection moulding using soft materials as described before. Materials may be selected to match the requirements of the drug delivery device, in particular the fluid-tightness at the pressure specified for the exit port. A particular advantage of this group of embodiments is that not only the rigid exit port sealing holder may be integrated into the housing, but the soft exit port sealing may also be combined with other sealings or functional elements requiring soft material on the same housing component. In figures 23a and 23b, the multiple sealing function of the soft exit port sealing is well visible: the sealing does not only cover the open distal end of the exit port channel 262a, but also comprises housing sealings 215 in the housing designed for other purposes. Channels of soft material may connect the different sealing elements to improve manufacturability. Within the limits of manufacturability any kind of housing component may be realised integrating the exit port assembly with other housing elements as described in this invention. Figure 23b is a cross section of the exit port assembly of figure 23a to show the shape of the soft exit port sealing 263 where it closes the distal end of the exit port channel 262a. Figure 23c shows a patch pump in an embodiment using the exit port assembly of figure 23a, in a fully assembled state, including the needle assembly 260 with the output portion 260b.

In a third group of embodiments according to the 6^{th} aspect of the present invention, the exit port is improved by keeping the soft exit port sealing as an own component and by improving manufacturability by introducing an exit port sealing plug cavity configured to allow easy insertion of the soft exit port sealing. With this approach there is no need to have an extra component as the rigid exit port sealing holder; the rigid exit port sealing holder is consequently integrated into a housing component.

Figure 24a-d illustrates a preferred embodiment of this group. Figure 24a shows the first housing component 213 in a perspective view (left) and in a cut across (right). The first housing component 213 is manufactured as one unitary component, for example by injection moulding and may also integrate other elements like housing sealings 215. The rigid exit port sealing holder 262 is an element of the first housing component 213, made of rigid material. The soft exit port sealing 263 is manufactured as a separate component, for example by injection moulding using a different mould. In this group of embodiments, the element of the first housing component corresponding the rigid exit port sealing holder 262 has an exit port sealing plug cavity 213c which is at least partially tubular and intersecting the exit port channel 262a at a minimal angle of 10 degrees, most preferably between 45 and 90 degrees. In figure 24a this is illustrated by indicating the exit port channel axis 262b and the exit port sealing plug cavity axis 213d. In this embodiment, the exit port sealing plug cavity 213c is open on an axial end. The soft exit port sealing may easily be inserted into the exit port sealing plug cavity 213c along the exit port sealing plug cavity axis 213d and provides a fluid-tight sealing of the exit port opening 213a. In this example, the exit port sealing has a substantially cylindrical shape, but may have any other shape suitable for easy inserting through an open face of the exit port sealing plug cavity while closing the exit port opening 213a in a fully assembled state. Two figures are added to further explain the shape of the exit port sealing plug cavity of this group of embodiments. Figure 24b is a perspective view of the first housing component 213 with rigid exit port sealing holder 262 cut along the exit port channel axis to show the shape of the exit port sealing plug cavity 213c. In figure 24c, the same view is shown, but with the soft exit port sealing inserted in the rigid exit port sealing cavity. In the example of figure 24a-d both the exit port sealing plug cavity 213c and the soft exit port sealing 263 have substantially the shape of a cylinder with a segment cut away. This shape is advantageous because the soft exit port sealing can be inserted into the rigid exit port sealing holder like a cylindrical plug while the flat side provides better fixation during the piercing step. The minimal angle between the exit port sealing plug cavity 213c and the axis of the exit port channel facilitates the manufacturing step of piercing the exit port sealing with the output portion 260b of the needle assembly 260. A further advantage of said angle between the exit port sealing plug cavity axis and the exit port channel axis is that when the output portion of the needle assembly is piercing through the soft exit port sealing the said soft exit port sealing is held in place by the rigid exit port sealing holder 262 with little or no shearing force or pulling force resulting at the connection between the soft exit port sealing and the rigid exit port sealing holder, again improving the sealing to reliably meet the requirements for fluid-tightness as specified.

Figure 24d shows a patch pump in an embodiment using the exit port assembly of figures 24a-c, in a fully assembled state, including the needle assembly 260 with the output portion 260b.

Figure 25a-e illustrates another preferred group of embodiments similar to the one in figure 24a-d. Figure 25a shows the first housing component 213 in a perspective view (left) and in a cut across (right). Like before, the first housing component 213 is manufactured as one unitary component, for example by injection moulding and may also integrate other elements like housing sealings 215. The rigid exit port sealing holder 262 is an element of the first housing component 213, made of rigid material. The soft exit port sealing 263 is manufactured as a separate component, for example by injection moulding using a different mould. Still like the group in figure 24a-d, the element of the first housing component corresponding the rigid exit port sealing holder 262 has an exit port sealing plug cavity 213c, filled in figure 25a with the soft exit port sealing 263. The exit port sealing plug cavity with exit port sealing plug cavity axis 213d is still intersecting the exit port channel 262a with exit port channel axis 262b at a minimal angle of 10 degrees, most preferably between 45 and 90 degrees. However, in this group of embodiments, the exit port sealing is no longer a stopper of a shape resembling a cylinder, but may have any other shape suitable to be pressed into the exit port sealing plug cavity through at least one open side. In this group of embodiments, the exit port sealing cavity axis does no longer imply a rotational symmetry, but indicates the path along which the soft exit port sealing is inserted into the rigid exit port seaing holder. To push a wide sealing a short way into a cavity - which can be seen as a sideways assembly of a stopper - is significantly easier than pushing a cylindrical sealing axially and brings a significant improvement to manufacturability. This group of embodiments has the additional advantage that the exit port sealing plug cavity can be designed with a big variety of shapes, in particular adding a constriction or flattening around the open distal end of the exit port channel to increase pressing and optimise fluid-tightness and pierceability in that area without compromising easy assembly during manufacturing. For sideways insertion the exit port sealing 263 is preferably not shaped like a cylinder, but more like a cushion or tablet, as shown in figure 25d (left). Two figures are added to further explain the shape of the exit port sealing plug cavity of this group of embodiments. Figure 25b is a perspective view of the first housing component 213 with rigid exit port sealing holder 262 cut along the exit port channel axis to show the shape of the exit port sealing plug cavity 213c. In figure 25c, the same view is shown, but with the soft exit port sealing inserted in the rigid exit port sealing cavity. Figure 25d shows the soft exit port sealing 263 before (left) and after (right) insertion into the exit port sealing plug cavity 262 of figure 25b. It is well visible that in the arrangement as described, the soft exit plug sealing 263 gets elastically deformed by this insertion and is compressed with a flat surface at a substantially orthogonal angle at the point where the output portion of the needle assembly will pierce the sealing.

Figure 25e shows a patch pump in an embodiment using the exit port assembly of figure 25ae, in a fully assembled state, including the needle assembly 260 with the output portion 260b.

Another set of preferred embodiments illustrates a 7^{th} aspect of the present invention. They are based on the wearable, semi-disposable patch pump shown in figures 1 and 2a,b as described before. They are further detailed in the following paragraphs.

The patch pump is further improved by covering the exit port chamber 213b with a semi-permeable exit port lid 285 removably attached to a component of the patch pump housing 214, as shown in figure 26a-c. The exit port lid 285 is configured to let air pass while any fluid, like for example water or a fluid medicament, is stopped and prevented from entering or leaving the exit port chamber 213b. With such an exit port lid, the needle assembly 260 and the reservoir 222 can be filled with medicament from the exterior without any of the fluid getting in contact with the patient. During filling of the reservoir 222 and/or priming of the fluid path through the needle assembly 260, the air initially present in the fluid path and/or in the exit port chamber is at least partially pressed through the exit port lid and replaced by fluid. The fluid, however, is stopped by the exit port lid. This has two advantageous effects: first, as mentioned before, no drug is spilled through the exit port while the exit port lid is in place; second, the exit port lid 285 closes the fluid path and ensures that a filling pressure may be applied to the fluid required to move the plunger 221 and fill the reservoir. By supporting the process of filling the reservoir and/or priming the fluid path, the exit port lid of this invention makes a big contribution to safe and reliable functioning of the drug delivery device. The exit port lid is realised using a priming membrane 285a made of a semi-permeable material, preferably in shape of a sheet, such as GoreTex^{®} or similar products. Because such membranes often are mechanically weak, a membrane reinforcing structure 285b is permanently fixed to the priming membrane 285a, preferably in the area surrounding the exit port chamber 213b, as shown in the basic embodiment of figure 26a. The membrane reinforcing structure may for example be a plastic ring, a grid, a net or a sheet of textile material strong enough to allow removal of the exit port lid without damaging the lid, and configured to allow contact of the fluid to the priming membrane. The membrane reinforcing structure 285b is removably attached to a housing component 214, for example by means of a glue or a double-sided adhesive tape. The double-sided adhesive tape may even be the same as the membrane reinforcing structure 285b, with a first side sticking permanently to the priming membrane 285a, and a second side removably sticking to a housing component 214. The presence of the membrane reinforcing structure is important to allow the exit port be designed for fluid-tightness at a specified minimum pressure such as the filling pressure, while still ensuring easy handling of the pump.

In this basic embodiment, the exit port lid 285 further includes an exit port lid liner 285c permanently fixed to the membrane reinforcing structure 285b, but not attached to the housing component 214. Using the exit port lid liner 286, the exit port lid 285 may easily be removed from the housing 214 after successful filling and/or priming of the patch pump. As filling and priming are not functions only present in patch pumps, the concept of an exit port lid may advantageously be applied to all kinds of drug delivery devices.

To achieve an optimum of accuracy and reliability, it is particularly important for patch pumps to keep the area around the output portion of the needle assembly well attached to the body of the patient during drug delivery, ideally with a fluid-tight connection. The exit port concept with the exit port lid is further improved by bringing the edge of the adhesive layer providing this fluid-tight connection closer to the exit port. This is realised by integrating the exit port lid into the adhesive patch assembly. A preferred example of such an embodiment is shown in figure 26b. The exit port lid 285 still includes the priming membrane 285a and the membrane reinforcing structure 285b. To bring the adhesive patch assembly 280 as close as possible to the border of the exit port chamber 213b a membrane carrying structure 212e is introduced. The membrane carrying structure is permanently attached to a housing component 214, preferably integrated therein as an element of the same unitary component. The membrane carrying structure has substantially the same thickness as the adhesive patch assembly 280 and is shaped to surround the exit port chamber in a way that a fluid-tight connection is made between the housing 214 and the exit port lid 285 as long as the exit port lid 285 is attached to the housing. A very simple example is a plastic ring integrated in or permanently attached to the housing component 214. In this embodiment, the adhesive patch assembly 280 has an adhesive layer 280a and an adhesive release liner 280b. With the membrane carrying structure 212e in place as described, the membrane reinforcing structure can remain in substantially the same plane while being removably attached to the membrane carrying structure 212e and permanently fixed to the adhesive release liner 280b. As before, the priming membrane 285a is permanently fixed to the membrane reinforcing structure 285b. This arrangement is easy to manufacture, may be optimised for fluid-tightness at a specified minimum pressure, brings further cost advantage by removing the need for a dedicated exit port lid liner (285c, see figure 26a) and brings about an optimum of ease of use. The exit port lid 285 is simply removed from the exit port chamber 213b by removing the adhesive release liner 280b from the adhesive layer 280a - which is a task the user has to do anyway to attach the patch pump to the body of the patient. Figure 26c shows a possible embodiment of an adhesive patch assembly 280 combined with an exit port lid 285 as described. The priming membrane 285a is mounted on the adhesive release liner 280b using a double-sided adhesive ring which also acts as a membrane reinforcing structure 285b. An adhesive mounting tape 280d is used to permanently attach the adhesive patch assembly 280 to the base plate 211a (not shown) at the bottom of the reservoir unit. A number of adhesive cut-outs 280c are introduced at least in the adhesive layer 280a, preferably cut through all layers of the adhesive patch assembly 280, to allow access to the pump housing for functions like filling the reservoir, bringing the needle assembly into position for drug delivery or pressure compensation.

Further improvements of the exit port lid in the scope of the present invention include a combination of the exit port lid and/or the membrane carrying structure with any other element on the pump housing or a combination of the exit port lid concept with other functions. As an example of such an extended function, the attachment of the exit port lid could be designed to break lose at low fluid pressure, deliberately allowing a leak to indicate to the user that the filling of the reservoir has been successfully completed, or to indicate to the user that a maximum pressure has been exceeded.

Another set of preferred embodiments illustrates an 8^{th} aspect of the present invention. They are based on the wearable, semi-disposable patch pump shown in figures 1 and 2a,b as described before. They are further detailed in the following paragraphs.

The patch pump is further improved by introducing, at the interface between the patch pump and the body of the patient, a number of airing channels. These airing channels allow air and humidity to get out of the interface into the environment. By doing so, the temperature and the humidity in the adhesive patch assembly and surrounding components is lowered, which improves the adherence of the patch pump to the body of the patient, and hence improves accuracy and reliability of the drug delivery. While similar airing channels have been known, a combination with other aspects of the present invention leads to a new solution, shown in figure 27. As described before, it is advantageous for the patch pump to keep the area around the output portion of the needle assembly well attached to the body of the patient during drug delivery, ideally with a fluid-tight connection. Therefore, in the embodiments of the present invention, the airing channels have no connection with the exit port chamber, but only start at a distance which is large enough for the adhesive patch assembly to ensure this fluid-tight connection, but small enough to improve adhesion in that area of the adhesive patch assembly. A preferred range of distance is 1 mm to 20 mm, most preferably 5 mm to 10 mm. In the preferred embodiment of figure 27, the airing channels are realised as ventilation grooves 212f in the base plate 211a of the reservoir unit 200. This example of an embodiment also includes the membrane carrying structure 212e. Accordingly, the adhesive layer covers the complete base plate 211a including base plate extension 212c, with a cut-out for the exit port and for the membrane carrying structure 212e. In figure 27 it is well visible, that the ventilation grooves 212f are arranged at a distance from the edge of the membrane carrying structure 212e. With the adhesive patch assembly (not shown) mounted on the base plate 211a, The ventilation grooves are covered by the adhesive patch assembly, creating airing channels which allow air and humidity to pass from the closed inner end 212g of the ventilation grooves 212f to the open outer end 212h of the ventilation grooves 212f and from there out into the environment in the exterior of the patch pump. In figure 27 it is also well visible that these airing channels have no connection with the exit port chamber 213b. It is evident that the shape or number of the airing channels is of no importance to their intended function. While the airing channels in the embodiment of figure 27 are realised as two substantially straight half cylinders, they may have any other geometry, size, arrangement or placing on the base plate 211a or the base plate extension 212c, as long as they have no connection with the exit port chamber 213b and at least one open end towards the environment in the exterior of the pump.

The result is a patch pump with improved airing at the interface between the bottom of the pump and the body of the patient, while still allowing an optimum of fluid-tight connection between the two sides of the interface around the exit port chamber, leading to improved accuracy and reliability of the drug delivery. Again, the arrangement of the present invention allows to design the housing of the pump and the adhesive patch assembly to achieve fluid-tightness at the minimum fluid pressure specified for this interface.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. Given the nature of the invention having a number of aspects contributing to an overall optimum, it is obvious to those skilled in the art that the improvements described, for better clarity, as different aspects, may be applied in any selection and/or combination. For example, a solution provided for an improved exit port sealing may well be used to improve the fill port or any other sealing in the drug delivery device. The fill port assembly may be combined with the exit port assembly to further optimise the pump. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF REFERENCE NUMERALS

- 1: Patch pump
- 1a: Enveloping surface
- 100: Pump unit
- 113: Locking mechanism
- 113a: Locking spring
- 114: Opening side
- 120: Drive mechanism
- 122: Plunger rod
- 123: Plunger rod cap
- 125: Threaded rod
- 140: System control circuitry
- 141: Printed circuit board, PCB-PU
- 142: Connecting pins
- 150: Rechargeable battery
- 151: Battery contact
- 200: Reservoir unit
- 211: Reservoir unit housing
- 211a: Base plate
- 211b: Wall
- 211c: Passage
- 211d: First recessed section
- 211e: Fixation pin
- 211f: Second recessed section
- 211g: Bottom wall
- 211h: Side wall
- 211i: Top wall
- 211j: Protruding rim
- 211k: Stabilising recess
- 211m: Cut-out
- 212a: Bayonet connection
- 212b: Rotation axis
- 212c: Base plate extension
- 212d: Locking structure
- 212e: Membrane carrying structure
- 212f: Ventilation groove
- 212g: Inner end
- 212h: Outer end
- 213: First housing component
- 213a: Exit port opening
- 213b: Exit port chamber
- 213c: Exit port sealing plug cavity
- 213d: Exit port sealing plug cavity axis
- 214: Housing component
- 215: Housing sealing
- 216: Pressure compensation membrane
- 221: Plunger
- 222: Reservoir
- 222a: Reservoir inlet
- 222b: Stabilising protrusion
- 222c: Reservoir outlet
- 222d: Reservoir outlet sealing cavity
- 222e: Reservoir axis
- 222f: Reinforcing ribs
- 222g: Fill port sealing cavity
- 223: Reservoir outlet sealing
- 230: Fill port assembly
- 231: Fill port sealing
- 231a: Flange
- 231b: Bore
- 231c: Pierceable septum
- 231d: Thermoplastic polymer
- 231e: Elastomer
- 231f: Cylindrical section
- 231g: Cone-shaped opening
- 235: Needle guide /Insert
- 235a: Cone shaped opening
- 235b: Base
- 235c: Sleeve
- 235d: Opening or cut-out
- 238: First sealing
- 239: Second sealing
- 240: Hybrid assembly
- 241: Base frame
- 243: Printed circuit board, PCB-RU
- 244: Battery
- 245: Battery opening
- 246: Retaining element
- 250: Inserter assembly
- 251: Cannula moving assembly
- 252: Insertion spring
- 253: Soft cannula holder
- 254: Rigid cannula holder
- 256: Insertion trigger
- 258: Rigid cannula
- 259: Soft cannula
- 259a: Soft cannula lumen
- 259b: Soft cannula sealing, input portion
- 260: Needle assembly
- 260a: Input portion
- 260b: Output portion
- 261: Exit port assembly
- 262: Rigid exit port sealing holder
- 262a: Exit port channel
- 262b: Exit port channel axis
- 263: Soft exit port sealing
- 270: Connector structure
- 271a-271d: Connector members
- 272: Contacting arm
- 272a: First electrical contact area
- 272b: Second electrical contact area
- 273: Switching arm
- 274: First battery contact arm
- 274a: First battery contact area
- 275: Second battery contact arm
- 275a: Second battery contact area
- 280: Adhesive patch assembly
- 280a: Adhesive layer
- 280b: Adhesive release liner
- 280c: Adhesive cut-out
- 285: Exit port lid
- 285a: Priming membrane
- 285b: Membrane reinforcing structure
- 285c: Exit port lid liner
- 290: Non-conductive body
- 291: First opening
- 292: Second opening
- 293: Fixation pins
- 294: Bearing pin
- 295: Upper guiding rail
- 296: Lower guiding rail
- 297: End stop surface
- 300: Patient

## Claims

1. A drug delivery device comprising a housing with an enveloping surface (1a) separating an interior volume from the exterior; a reservoir (222) located inside the housing to contain a drug; a needle assembly (260) with an input portion (260a) and an output portion (260b); and an exit port assembly (261), whereby
the housing comprises at least two components (213, 214), configured to be attached to one another to form a protective shell of the drug delivery device;
the reservoir (222) comprises a reservoir outlet (222c) coupled to the input portion (260a) of the needle assembly (260);
the housing comprises an exit port opening (213a) to provide a passage for the output portion (260b) of the needle assembly (260) from the interior of the housing to the exterior;
the exit port assembly (261) is configured to form a fluid-tight connection between the housing and the output portion of the needle assembly (260);
the exit port assembly (261) comprises a rigid exit port sealing holder (262) with an at least partially tubular exit port channel (262a) defining an exit port channel axis (262b) configured to receive at least a portion of the needle assembly (260); and a soft exit port sealing (263) attached to the rigid exit port sealing holder (262);
the needle assembly (260) is movable from an initial position for storage to an inserted position for drug delivery;
the exit port assembly (261) is fixedly attached to at least one component of the housing;
the soft exit port sealing (263) is configured to provide a fluid-tight closure of the exit port opening (213a) with the output portion (260b) of the needle assembly (260) located on the distal side of the soft exit port sealing both in the initial position and in the inserted position of the needle assembly (260);
***characterised by***
the rigid exit port sealing holder (262) further includes an exit port sealing plug cavity (213c) defining an exit port sealing plug cavity axis (213d), and open on at least one end to receive the soft exit port sealing (263);
the exit port sealing plug cavity (213c) is arranged to intersect the exit port channel (262a) with an angle of at least 10 degrees, preferably at least 45 degrees between the exit port channel axis (262b) and the exit port sealing plug cavity axis (213d);
the soft exit port sealing (263) is mounted into the exit port sealing plug cavity (213c), for example by applying a press-fit technology, and tightly closing the exit port sealing plug cavity (213c) with the needle assembly (260) pierced through and the output portion (260b) of the needle assembly (260) located on the distal side of the soft exit port sealing both in the initial position and in the inserted position of the needle assembly (260).

2. A drug delivery device according to claim 1, where the exit port assembly (261) is a 2-shot injection moulded unitary component.

3. A drug delivery device according to claim 1 or 2, whereby the exit port assembly (261) is manufactured using a 2-shot injection moulding process as a unitary component with one of the at least two housing components (213, 214).

4. A drug delivery device according to claim 1, whereby
the exit port sealing plug cavity (213c) has a constriction and/or flattening at the exit port opening (213a) to improve pressing and/or shaping of the soft exit port sealing (263) at the intersection with the exit port channel (262a).

5. A drug delivery device according to any of the preceding claims, whereby
the housing has a recess from the enveloping surface of the housing at the exit port opening;
the recess in the housing forms an external exit port chamber (213b) between the recessed exit port opening (213a) and the enveloping surface (1a) of the housing;

6. A drug delivery device according to any of the preceding claims, whereby the needle assembly (260) comprises a soft cannula (259) having an open distal end, a rigid cannula (258) at least partially and slidably disposed in an inner soft cannula lumen (259a) of the soft cannula, and a needle insertion mechanism configured to bring the output portion (260b) of the needle assembly (260) with at a least the open distal end of the soft cannula from a first position inside the exit port chamber to a second position outside the exit port chamber in the exterior of the housing.

7. A drug delivery system according to any of the preceding claims, further comprising an exit port lid (285), whereby
the exit port lid (285) is removably attached to the housing at a portion of the housing surrounding the exit port opening;
the exit port lid (285) covers the exit port chamber (213b) in a way that prevents fluid from entering or leaving the exit port chamber (213b) while letting air pass;
the exit port lid (285) includes a membrane of any semi-permeable material adapted to allow air to pass through while stopping liquid from passing;
the exit port lid (285) includes a membrane reinforcing structure (285b) permanently fixed to the membrane to ensure the membrane is not damaged in the process of removing the exit port lid (285) from the housing.

8. A drug delivery system according to claim 7 whereby the membrane reinforcing structure (285b) includes a plastic sheet with a cut-out for the exit port opening.

9. A drug delivery device according to claim 7 or 8 configured to be used as a patch pump with an adhesive patch assembly (280) to attach the patch pump (1) to a patient (300), whereby
the adhesive patch assembly (280) comprises an adhesive layer configured to attach the adhesive patch assembly (280) to the patient (300) after preparation of the patch pump (1) for drug delivery;
the adhesive patch assembly (280) comprises a removable adhesive release liner to protect the adhesive layer prior to use, in particular during preparation of the patch pump (1) for drug delivery;
the exit port lid (285) is fixed permanently to the adhesive release liner and removably connected to the housing; and
the exit port lid (285) is configured to be removed from the housing together with the adhesive release liner during preparation of the patch pump (1) for drug delivery.

10. A patch pump (1) according to claim 9, whereby
the housing further comprises a membrane carrying structure arranged around the exit port chamber;
the membrane carrying structure is adapted to protrude from the housing towards the exterior by a height substantially the same as the thickness of the adhesive patch assembly;
the membrane carrying structure is configured to provide contact with the exit port lid (285) in a status where the exit port lid (285) is removably attached to the housing;
the membrane carrying structure is configured to provide a fluid-tight connection between the exit port lid (285) and the housing during preparation of the patch pump (1) for drug delivery and thereby tightly close the exit port chamber.

11. A patch pump (1) according to claims 9 or 10, whereby
the housing and/or the adhesive patch assembly further comprises at least one ventilation structure with at least one inner end closed and arranged at a horizontal distance of 1 mm to 20 mm, preferably 5 mm to 10 mm from the exit port chamber, and at least one outer end left open and arranged at the peripheral edge of the housing and/or the adhesive;
the ventilation structure is configured to let air pass from the inner end to the outer end and to the environment of the patch pump (1).

12. A method of manufacturing a drug delivery device according to claim 1,
the method comprising, without implying any particular order or separation, the steps of
- manufacturing the exit port assembly (261) by attaching the soft exit port sealing (263) to the rigid exit port sealing holder (262), for example by using 2-shot injection moulding technology, by gluing, by press-fitting or any other technology establishing a fluid-tight connection between the two components;
- attaching the rigid exit port sealing holder (262) to at least one housing component; and
- piercing the soft exit port sealing (263) with the output portion (260b) of the needle assembly (260) to establish an initial position.

## Patentansprüche

1. Medikamentenabgabevorrichtung umfassend ein Gehäuse mit einer Umhüllungsfläche (1a), die ein Innenvolumen von der Außenseite trennt; ein Reservoir (222), das innerhalb des Gehäuses angeordnet ist, um ein Medikament aufzunehmen; eine Nadelanordnung (260) mit einem Eingangsabschnitt (260a) und einem Ausgangsabschnitt (260b); und eine Ausgangsöffnungsbaugruppe (261), wobei
das Gehäuse mindestens zwei Komponenten (213, 214) umfasst, die so konfiguriert sind, dass sie aneinander befestigt werden können, um eine Schutzhülle der Medikamentenabgabevorrichtung zu bilden;
das Reservoir (222) einen Reservoirauslass (222c) umfasst, der mit dem Eingangsabschnitt (260a) der Nadelanordnung (260) gekoppelt ist;
das Gehäuse eine Ausgangsöffnung (213a) umfasst, um einen Durchgang für den Ausgangsabschnitt (260b) der Nadelanordnung (260) vom Inneren des Gehäuses nach außen bereitzustellen;
die Ausgangsöffnungsbaugruppe (261) so konfiguriert ist, dass sie eine fluiddichte Verbindung zwischen dem Gehäuse und dem Ausgangsabschnitt der Nadelanordnung (260) bildet;
die Ausgangsöffnungsbaugruppe (261) einen starren Ausgangsöffnungsdichtungshalter (262) mit einem zumindest teilweise rohrförmigen Ausgangsöffnungskanal (262a) umfasst, der eine Ausgangsöffnungskanalachse (262b) definiert, die so konfiguriert ist, dass sie mindestens einen Teil der Nadelanordnung (260) aufnimmt; und eine weiche Ausgangsöffnungsdichtung (263), die an dem starren Ausgangsöffnungsdichtungshalter (262) befestigt ist;
die Nadelanordnung (260) von einer Ausgangsposition zur Lagerung in eine eingeführte Position zur Medikamentenabgabe bewegbar ist;
die Ausgangsöffnungsbaugruppe (261) fest an mindestens einer Komponente des Gehäuses befestigt ist;
die weiche Ausgangsöffnungsdichtung (263) so konfiguriert ist, dass sie einen fluiddichten Verschluss der Ausgangsöffnung (213a) bereitstellt, wobei der Ausgangsabschnitt (260b) der Nadelanordnung (260) auf der distalen Seite der weichen Ausgangsöffnungsdichtung sowohl in der Ausgangsposition als auch in der eingeführten Position der Nadelanordnung (260) angeordnet ist;
***dadurch gekennzeichnet, dass***
der starre Ausgangsöffnungsdichtungshalter (262) ferner einen Ausgangsöffnungsdichtungsstopfenhohlraum (213c) umfasst, der eine Ausgangsöffnungsdichtungsstopfenhohlraumachse (213d) definiert und an mindestens einem Ende offen ist, um die weiche Ausgangsöffnungsdichtung (263) aufzunehmen;
der Ausgangsöffnungsdichtungsstopfenhohlraum (213c) so angeordnet ist, dass er den Ausgangsöffnungskanal (262a) mit einem Winkel von mindestens 10 Grad, vorzugsweise mindestens 45 Grad, zwischen der Ausgangsöffnungskanalachse (262b) und der Ausgangsöffnungsdichtungsstopfenhohlraumachse (213d) schneidet;
die weiche Ausgangsöffnungsdichtung (263) in den Ausgangsöffnungsdichtungsstopfenhohlraum (213c) eingesetzt ist, beispielsweise durch Anwenden einer Einpresstechnologie, und den Ausgangsöffnungsdichtungsstopfenhohlraum (213c) dicht verschließt, wobei die Nadelanordnung (260) hindurchgestochen ist und der Ausgangsabschnitt (260b) der Nadelanordnung (260) auf der distalen Seite der weichen Ausgangsöffnungsdichtung sowohl in der Ausgangsposition als auch in der eingeführten Position der Nadelanordnung (260) angeordnet ist.

2. Medikamentenabgabevorrichtung nach Anspruch 1, wobei die Ausgangsöffnungsbaugruppe (261) ein durch 2-Komponenten-Spritzguss hergestelltes einstückiges Bauteil ist.

3. Medikamentenabgabevorrichtung nach Anspruch 1 oder 2, wobei die Ausgangsöffnungsbaugruppe (261) mittels eines 2-Komponenten-Spritzgussverfahrens als einstückiges Bauteil mit einer der mindestens zwei Gehäusekomponenten (213, 214) hergestellt ist.

4. Medikamentenabgabevorrichtung nach Anspruch 1, wobei
der Ausgangsöffnungsdichtungsstopfenhohlraum (213c) eine Verengung und/oder Abflachung an der Ausgangsöffnung (213a) aufweist, um das Pressen und/oder Formen der weichen Ausgangsöffnungsdichtung (263) am Schnittpunkt mit dem Ausgangsöffnungskanal (262a) zu verbessern.

5. Medikamentenabgabevorrichtung nach einem der vorhergehenden Ansprüche, wobei
das Gehäuse eine Vertiefung von der Umhüllungsfläche des Gehäuses an der Ausgangsöffnung aufweist;
die Vertiefung im Gehäuse eine externe Ausgangsöffnungskammer (213b) zwischen der vertieften Ausgangsöffnung (213a) und der Umhüllungsfläche (1a) des Gehäuses bildet;

6. Medikamentenabgabevorrichtung nach einem der vorhergehenden Ansprüche, wobei die Nadelanordnung (260) eine weiche Kanüle (259) mit einem offenen distalen Ende, eine starre Kanüle (258), die zumindest teilweise und gleitend in einem inneren Weichkanülenlumen (259a) der weichen Kanüle angeordnet ist, und einen Nadeleinführmechanismus umfasst, der so konfiguriert ist, dass er den Ausgangsabschnitt (260b) der Nadelanordnung (260) mit mindestens dem offenen distalen Ende der weichen Kanüle von einer ersten Position innerhalb der Ausgangsöffnungskammer in eine zweite Position außerhalb der Ausgangsöffnungskammer außerhalb des Gehäuses bringt.

7. Medikamentenabgabesystem nach einem der vorhergehenden Ansprüche, ferner umfassend einen Ausgangsöffnungsdeckel (285), wobei
der Ausgangsöffnungsdeckel (285) abnehmbar an dem Gehäuse an einem Abschnitt des Gehäuses befestigt ist, der die Ausgangsöffnung umgibt;
der Ausgangsöffnungsdeckel (285) die Ausgangsöffnungskammer (213b) so abdeckt, dass verhindert wird, dass Flüssigkeit in die Ausgangsöffnungskammer (213b) eintritt oder diese verlässt, während Luft hindurchgelassen wird;
der Ausgangsöffnungsdeckel (285) eine Membran aus einem beliebigen semipermeablen Material umfasst, das so angepasst ist, dass Luft hindurchgelassen wird, während Flüssigkeit am Durchgang gehindert wird;
der Ausgangsöffnungsdeckel (285) eine Membranverstärkungsstruktur (285b) umfasst, die dauerhaft an der Membran befestigt ist, um sicherzustellen, dass die Membran beim Entfernen des Ausgangsöffnungsdeckels (285) vom Gehäuse nicht beschädigt wird.

8. Medikamentenabgabesystem nach Anspruch 7, wobei die Membranverstärkungsstruktur (285b) eine Kunststofffolie mit einem Ausschnitt für die Ausgangsöffnung umfasst.

9. Medikamentenabgabevorrichtung nach Anspruch 7 oder 8, die zur Verwendung als Pflasterpumpe mit einer Klebepflasterbaugruppe (280) zum Anbringen der Pflasterpumpe (1) an einem Patienten (300) konfiguriert ist, wobei
die Klebepflasterbaugruppe (280) eine Klebeschicht umfasst, die so konfiguriert ist, dass die Klebepflasterbaugruppe (280) nach Vorbereitung der Pflasterpumpe (1) zur Medikamentenabgabe am Patienten (300) befestigt wird;
die Klebepflasterbaugruppe (280) eine abnehmbare Klebefreigabefolie zum Schutz der Klebeschicht vor der Verwendung umfasst, insbesondere während der Vorbereitung der Pflasterpumpe (1) zur Medikamentenabgabe;
der Ausgangsöffnungsdeckel (285) dauerhaft an der Klebefreigabefolie befestigt und abnehmbar mit dem Gehäuse verbunden ist; und
der Ausgangsöffnungsdeckel (285) so konfiguriert ist, dass er zusammen mit der Klebefreigabefolie während der Vorbereitung der Pflasterpumpe (1) zur Medikamentenabgabe vom Gehäuse entfernt wird.

10. Pflasterpumpe (1) nach Anspruch 9, wobei
das Gehäuse ferner eine Membranträgerstruktur umfasst, die um die Ausgangsöffnungskammer herum angeordnet ist;
die Membranträgerstruktur so angepasst ist, dass sie vom Gehäuse nach außen um eine Höhe vorsteht, die im Wesentlichen der Dicke der Klebepflasterbaugruppe entspricht;
die Membranträgerstruktur so konfiguriert ist, dass sie einen Kontakt mit dem Ausgangsöffnungsdeckel (285) in einem Zustand bereitstellt, in dem der Ausgangsöffnungsdeckel (285) abnehmbar am Gehäuse befestigt ist;
die Membranträgerstruktur so konfiguriert ist, dass sie eine fluiddichte Verbindung zwischen dem Ausgangsöffnungsdeckel (285) und dem Gehäuse während der Vorbereitung der Pflasterpumpe (1) zur Medikamentenabgabe bereitstellt und dadurch die Ausgangsöffnungskammer dicht verschließt.

11. Pflasterpumpe (1) nach Anspruch 9 oder 10, wobei
das Gehäuse und/oder die Klebepflasterbaugruppe ferner mindestens eine Belüftungsstruktur mit mindestens einem inneren Ende umfasst, das geschlossen und in einem horizontalen Abstand von 1 mm bis 20 mm, vorzugsweise 5 mm bis 10 mm, von der Ausgangsöffnungskammer angeordnet ist, und mindestens einem äußeren Ende, das offen gelassen und am peripheren Rand des Gehäuses und/oder des Klebepflasters angeordnet ist;
die Belüftungsstruktur so konfiguriert ist, dass sie Luft vom inneren Ende zum äußeren Ende und zur Umgebung der Pflasterpumpe (1) durchlässt.

12. Verfahren zur Herstellung einer Medikamentenabgabevorrichtung nach Anspruch 1, wobei das Verfahren, ohne eine bestimmte Reihenfolge oder Trennung zu implizieren, die folgenden Schritte umfasst:
- Herstellen der Ausgangsöffnungsbaugruppe (261) durch Befestigen der weichen Ausgangsöffnungsdichtung (263) an dem starren Ausgangsöffnungsdichtungshalter (262), beispielsweise unter Verwendung von 2-Komponenten-Spritzgusstechnologie, durch Kleben, durch Einpressen oder jede andere Technologie, die eine fluiddichte Verbindung zwischen den beiden Komponenten herstellt;
- Befestigen des starren Ausgangsöffnungsdichtungshalters (262) an mindestens einer Gehäusekomponente; und
- Durchstechen der weichen Ausgangsöffnungsdichtung (263) mit dem Ausgangsabschnitt (260b) der Nadelanordnung (260), um eine Ausgangsposition herzustellen.

## Revendications

1. Dispositif d'administration de médicament comprenant un boîtier avec une surface enveloppante (1a) séparant un volume intérieur de l'extérieur ; un réservoir (222) situé à l'intérieur du boîtier pour contenir un médicament ; un ensemble d'aiguille (260) avec une partie d'entrée (260a) et une partie de sortie (260b) ; et un ensemble d'orifice de sortie (261), dans lequel
le boîtier comprend au moins deux composants (213, 214), configurés pour être fixés l'un à l'autre afin de former une coque de protection du dispositif d'administration de médicament;
le réservoir (222) comprend une sortie de réservoir (222c) couplée à la partie d'entrée (260a) de l'ensemble d'aiguille (260) ;
le boîtier comprend une ouverture d'orifice de sortie (213a) pour fournir un passage pour la partie de sortie (260b) de l'ensemble d'aiguille (260) de l'intérieur du boîtier vers l'extérieur ;
l'ensemble d'orifice de sortie (261) est configuré pour former une connexion étanche aux fluides entre le boîtier et la partie de sortie de l'ensemble d'aiguille (260) ;
l'ensemble d'orifice de sortie (261) comprend un support d'étanchéité d'orifice de sortie rigide (262) avec un canal d'orifice de sortie au moins partiellement tubulaire (262a) définissant un axe de canal d'orifice de sortie (262b) configuré pour recevoir au moins une partie de l'ensemble d'aiguille (260) ; et une étanchéité d'orifice de sortie souple (263) fixée au support d'étanchéité d'orifice de sortie rigide (262) ;
l'ensemble d'aiguille (260) est mobile d'une position initiale pour le stockage à une position insérée pour l'administration de médicament ;
l'ensemble d'orifice de sortie (261) est fixé de manière fixe à au moins un composant du boîtier;
l'étanchéité d'orifice de sortie souple (263) est configurée pour fournir une fermeture étanche aux fluides de l'ouverture d'orifice de sortie (213a) avec la partie de sortie (260b) de l'ensemble d'aiguille (260) située sur le côté distal de l'étanchéité d'orifice de sortie souple aussi bien dans la position initiale que dans la position insérée de l'ensemble d'aiguille (260) ;
***caractérisé en ce que***
le support d'étanchéité d'orifice de sortie rigide (262) comprend en outre une cavité de bouchon d'étanchéité d'orifice de sortie (213c) définissant un axe de cavité de bouchon d'étanchéité d'orifice de sortie (213d), et ouverte sur au moins une extrémité pour recevoir l'étanchéité d'orifice de sortie souple (263) ;
la cavité de bouchon d'étanchéité d'orifice de sortie (213c) est agencée pour croiser le canal d'orifice de sortie (262a) avec un angle d'au moins 10 degrés, de préférence d'au moins 45 degrés entre l'axe de canal d'orifice de sortie (262b) et l'axe de cavité de bouchon d'étanchéité d'orifice de sortie (213d) ;
l'étanchéité d'orifice de sortie souple (263) est montée dans la cavité de bouchon d'étanchéité d'orifice de sortie (213c), par exemple en appliquant une technologie d'emmanchement à force, et fermant hermétiquement la cavité de bouchon d'étanchéité d'orifice de sortie (213c) avec l'ensemble d'aiguille (260) percé à travers et la partie de sortie (260b) de l'ensemble d'aiguille (260) située sur le côté distal de l'étanchéité d'orifice de sortie souple aussi bien dans la position initiale que dans la position insérée de l'ensemble d'aiguille (260).

2. Dispositif d'administration de médicament selon la revendication 1, dans lequel l'ensemble d'orifice de sortie (261) est un composant unitaire moulé par injection bi-matière.

3. Dispositif d'administration de médicament selon la revendication 1 ou 2, dans lequel l'ensemble d'orifice de sortie (261) est fabriqué en utilisant un procédé de moulage par injection bi-matière en tant que composant unitaire avec l'un des au moins deux composants de boîtier (213, 214).

4. Dispositif d'administration de médicament selon la revendication 1, dans lequel
la cavité de bouchon d'étanchéité d'orifice de sortie (213c) présente un rétrécissement et/ou un aplatissement à l'ouverture d'orifice de sortie (213a) pour améliorer le pressage et/ou le façonnage de l'étanchéité d'orifice de sortie souple (263) à l'intersection avec le canal d'orifice de sortie (262a).

5. Dispositif d'administration de médicament selon l'une quelconque des revendications précédentes, dans lequel
le boîtier présente un renfoncement à partir de la surface enveloppante du boîtier au niveau de l'ouverture d'orifice de sortie ;
le renfoncement dans le boîtier forme une chambre d'orifice de sortie externe (213b) entre l'ouverture d'orifice de sortie en renfoncement (213a) et la surface enveloppante (1a) du boîtier ;

6. Dispositif d'administration de médicament selon l'une quelconque des revendications précédentes, dans lequel l'ensemble d'aiguille (260) comprend une canule souple (259) ayant une extrémité distale ouverte, une canule rigide (258) au moins partiellement et de manière coulissante disposée dans une lumière de canule souple interne (259a) de la canule souple, et un mécanisme d'insertion d'aiguille configuré pour amener la partie de sortie (260b) de l'ensemble d'aiguille (260) avec au moins l'extrémité distale ouverte de la canule souple d'une première position à l'intérieur de la chambre d'orifice de sortie à une seconde position à l'extérieur de la chambre d'orifice de sortie dans l'extérieur du boîtier.

7. Système d'administration de médicament selon l'une quelconque des revendications précédentes, comprenant en outre un couvercle d'orifice de sortie (285), dans lequel
le couvercle d'orifice de sortie (285) est fixé de manière amovible au boîtier au niveau d'une partie du boîtier entourant l'ouverture d'orifice de sortie ;
le couvercle d'orifice de sortie (285) couvre la chambre d'orifice de sortie (213b) d'une manière qui empêche le fluide d'entrer ou de sortir de la chambre d'orifice de sortie (213b) tout en laissant passer l'air ;
le couvercle d'orifice de sortie (285) comprend une membrane de tout matériau semi-perméable adapté pour permettre à l'air de passer tout en empêchant le liquide de passer ;
le couvercle d'orifice de sortie (285) comprend une structure de renforcement de membrane (285b) fixée de manière permanente à la membrane pour garantir que la membrane n'est pas endommagée lors du processus de retrait du couvercle d'orifice de sortie (285) du boîtier.

8. Système d'administration de médicament selon la revendication 7, dans lequel la structure de renforcement de membrane (285b) comprend une feuille de plastique avec une découpe pour l'ouverture d'orifice de sortie.

9. Dispositif d'administration de médicament selon la revendication 7 ou 8 configuré pour être utilisé comme une pompe patch avec un ensemble de patch adhésif (280) pour fixer la pompe patch (1) à un patient (300), dans lequel
l'ensemble de patch adhésif (280) comprend une couche adhésive configurée pour fixer l'ensemble de patch adhésif (280) au patient (300) après la préparation de la pompe patch (1) pour l'administration de médicament ;
l'ensemble de patch adhésif (280) comprend un film protecteur adhésif amovible pour protéger la couche adhésive avant utilisation, en particulier pendant la préparation de la pompe patch (1) pour l'administration de médicament ;
le couvercle d'orifice de sortie (285) est fixé de manière permanente au film protecteur adhésif et connecté de manière amovible au boîtier ; et
le couvercle d'orifice de sortie (285) est configuré pour être retiré du boîtier ensemble avec le film protecteur adhésif pendant la préparation de la pompe patch (1) pour l'administration de médicament.

10. Pompe patch (1) selon la revendication 9, dans laquelle
le boîtier comprend en outre une structure porteuse de membrane agencée autour de la chambre d'orifice de sortie ;
la structure porteuse de membrane est adaptée pour faire saillie du boîtier vers l'extérieur d'une hauteur sensiblement égale à l'épaisseur de l'ensemble de patch adhésif ;
la structure porteuse de membrane est configurée pour fournir un contact avec le couvercle d'orifice de sortie (285) dans un état où le couvercle d'orifice de sortie (285) est fixé de manière amovible au boîtier ;
la structure porteuse de membrane est configurée pour fournir une connexion étanche aux fluides entre le couvercle d'orifice de sortie (285) et le boîtier pendant la préparation de la pompe patch (1) pour l'administration de médicament et fermer ainsi hermétiquement la chambre d'orifice de sortie.

11. Pompe patch (1) selon la revendication 9 ou 10, dans laquelle
le boîtier et/ou l'ensemble de patch adhésif comprend en outre au moins une structure de ventilation avec au moins une extrémité interne fermée et agencée à une distance horizontale de 1 mm à 20 mm, de préférence de 5 mm à 10 mm de la chambre d'orifice de sortie, et au moins une extrémité externe laissée ouverte et agencée au bord périphérique du boîtier et/ou de l'adhésif ;
la structure de ventilation est configurée pour laisser passer l'air de l'extrémité interne à l'extrémité externe et vers l'environnement de la pompe patch (1).

12. Procédé de fabrication d'un dispositif d'administration de médicament selon la revendication 1, le procédé comprenant, sans impliquer un ordre ou une séparation particulier, les étapes de
- fabrication de l'ensemble d'orifice de sortie (261) en fixant l'étanchéité d'orifice de sortie souple (263) au support d'étanchéité d'orifice de sortie rigide (262), par exemple en utilisant une technologie de moulage par injection bi-matière, par collage, par emmanchement à force ou toute autre technologie établissant une connexion étanche aux fluides entre les deux composants ;
- fixation du support d'étanchéité d'orifice de sortie rigide (262) à au moins un composant de boîtier ; et
- perçage de l'étanchéité d'orifice de sortie souple (263) avec la partie de sortie (260b) de l'ensemble d'aiguille (260) pour établir une position initiale.
